(19) Europäisches Patentamt
European Patent Office
Office européen des brevets

(11) EP 3 453 384 B1

(12) # EUROPEAN PATENT SPECIFICATION

(45) Date of publication and mention of the grant of the patent:
**27.05.2020 Bulletin 2020/22**

(51) Int Cl.:
***A61K 9/20*** *(2006.01)* ***A61K 31/445*** *(2006.01)*

(21) Application number: **17208871.8**

(22) Date of filing: **20.12.2017**

(54) **PHARMACEUTICAL TABLET COMPOSITION COMPRISING BILASTINE**

PHARMAZEUTISCHE TABLETTE ENHALTEND BILASTIN

COMPRIME PHARMACEUTIQUE COMPRENANT DU BILASTINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **07.09.2017 IN 201711031743**

(43) Date of publication of application:
**13.03.2019 Bulletin 2019/11**

(73) Proprietor: **Alfred E. Tiefenbacher (GmbH & Co. KG)**
**22767 Hamburg (DE)**

(72) Inventors:
- **Gujjar, Chaitanya Yogananda**
**502 319 Telangana (IN)**
- **Uppala, Susheel Prakash**
**502 319 Telangana (IN)**
- **Donga, Nani Prasad**
**502 319 Telangana (IN)**
- **Bandla, Srimannarayana**
**502 319 Telangana (IN)**
- **Rallabandi, Bala Ramesha Chary**
**502 319 Telangana (IN)**
- **Staver, Ruslan**
**22607 Hamburg (DE)**
- **Schlehahn, Hendrik**
**23843 Travenbrueck (DE)**

(74) Representative: **Hertin und Partner**
**Rechts- und Patentanwälte PartG mbB**
**Kurfürstendamm 54/55**
**10707 Berlin (DE)**

(56) References cited:
**EP-A1- 1 505 066**
**WO-A1-2017/017301**
**WO-A2-2014/026657**
**CN-A- 103 356 616**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 453 384 B1

## Description

The invention relates to a pharmaceutical composition in the form of a tablet, comprising a) a crystalline form of bilastine according to polymorph form 2, wherein the crystalline form has characteristic peaks at 6.53, 15.24, 15.86, and 18.07 ± 0.2 degrees 2-theta in a powder X-ray diffraction pattern, b) a water-soluble filler, and optionally c) a water-insoluble filler, wherein the water-soluble filler is a sugar, and wherein the composition is prepared by dry granulation of a blend of the components comprising a), b) and optionally c), and compression of the granules to a tablet, or direct compression of a blend of the components comprising a), b) and optionally c). The invention further relates to a method of preparing a pharmaceutical composition in the form of a tablet comprising dry granulation of a blend of the tablet components and compression of the granules to a tablet, or direct compression of a blend to a tablet. The invention relates further to the medical use of the pharmaceutical composition in the treatment of allergic rhino-conjunctivitis and/or urticaria.

## BACKGROUND OF THE INVENTION

Bilastine (INN) is known under the chemical name 2-[4-(2-(4-(1-(2-ethoxyethyl)-1 H-benzimidazol-2-yl)piperidin-1-yl)ethyl)phenyl]-2-methylpropionic acid and CAS number 202189-78-4. It has the following chemical structure:

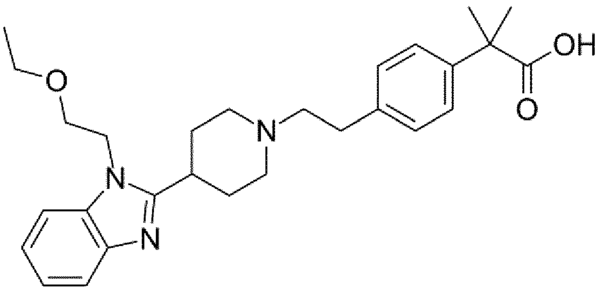

Bilastine is a non-sedating, long-acting histamine antagonist with selective peripheral $H_1$ receptor antagonist affinity and no affinity for muscarinic receptors. Its effectiveness is similar to cetirizine, fexofenadine and desloratadine. Bilastine can be classified into the same chemical group as many of the new antihistamines on the market, although it is not structurally derived, nor is it a metabolite or enantiomer of any of them, but an original molecule designed with the intent of fulfilling all the requirements of a second-generation antihistamine.

It was developed by FAES Farma (former name: Fabrica Espanola de Productos Quimicos y Farmaceuticos SA). Bilastine is approved in the EU for 20 mg once daily dosing for the relief of symptoms of allergic rhino conjunctivitis and urticaria in adults and adolescents. Bilastine is marketed by FAES and its European licensing partner Menarini under different tradenames, e.g. as Bilaska in France and as Bitosen in Germany. Bilastine was first disclosed in EP0818454.

EP1505066 discloses a polymorphic form of bilastine, described as polymorphic form 1. According to EP1505066, bilastine can exist in three different polymorphic forms, called polymorph 1, polymorph 2 and polymorph 3. The procedure described in EP818454 generates a mixture of polymorphs 2 and 3. Polymorph 2, polymorph 3, and their mixture are subsequently converted into polymorph 1 by the procedures of EP1505066, as shown in examples 1-5.

The public assessment report (PAR) of BfArM for 20 mg bilastine tablets of FAES discloses similar information, namely that three polymorphic forms have been identified during development of bilastine by FAES and the active substance consists of polymorphic form 1. Furthermore, the PAR discloses that FAES manufactures tablets by using direct compression of bilastine with the following excipients: microcrystalline cellulose, sodium carboxymethyl starch, anhydrous colloidal silica and magnesium stearate.

WO 2014/026657 (Zentiva) discloses the preparation of polymorphic forms 1 and 2 of bilastine and discloses not only IR data, but also XRD patterns. No formulations or particular pharmaceutical compositions are disclosed.

WO 2017/017301 (Urquima) discloses the preparation of novel hydrate forms of bilastine, for examples forms Alpha and Eta, as well as the preparation of polymorphic forms 1, 2, and 3 and their XRD, IR, and DSC data. According to WO 2017/017301, the neat polymorphic forms 1 and 2 of bilastine and hydrates Alpha and Eta are stable on storage. Example 10 of WO 2017/017301 discloses pharmaceutical formulations using the hydrate forms Alpha and Eta and with neat form 2 (polymorphic form 2). The formulation with form 2 contains the same excipients as the reference product, as described in the PAR, mentioned above.

Despite the provision of various polymorph forms of bilastine and pharmaceutical compositions comprising the same, improvements are required for improved or more efficient means of formulating bilastine using a simplified and

reliable method to provide stable formulations for medical administration.

## SUMMARY OF THE INVENTION

In light of the prior art the technical problem underlying the invention was the provision of improved or alternative means for pharmaceutical compositions comprising bilastine that do not exhibit the disadvantages of the prior art. A further objective of the invention was the provision of simplified means for formulating bilastine, preferably the polymorph form 2 of bilastine, in a stable composition.

This problem is solved by the features of the independent claims. Preferred embodiments of the present invention are provided by the dependent claims.

The invention therefore relates to a pharmaceutical composition in the form of a tablet, comprising a) a crystalline form of bilastine, wherein the crystalline form has characteristic peaks at 6.53, 15.24, 15.86, and 18.07 $\pm$ 0.2 degrees 2-theta in a powder X-ray diffraction pattern, b) a water-soluble filler, and optionally c) a water-insoluble filler, wherein the water-soluble filler is a sugar, and wherein the composition is prepared by dry granulation of a blend of the components comprising a), b) and optionally c), and compression of the granules to a tablet, or direct compression of a blend of the components comprising a), b) and optionally c).

In one embodiment of the invention, the pharmaceutical composition is characterized in that said composition is prepared by dry granulation of a blend of the components a), b) and optionally c) and compression of the granules to a tablet. In a preferred embodiment, the method is characterized by blending the components a), b) and optionally c), roller compaction and granulation of the blend, milling the granules, lubricating the granules and subsequent compression of the granules to a tablet.

The dry granulation of the blend represents an established, cost-efficient, reliable and high-throughput approach towards formulation, which represents an improvement in efficiency and reliability over alternative methods of production, for example in comparison to wet granulation followed by a subsequent tabletting. In one embodiment, the dry granulation of the present invention further represents an inherent description of the structural features of the tablet composition.

In one embodiment of the invention, the pharmaceutical composition is characterized in that the composition is prepared by direct compression of a powder blend of the components of a), b) and optionally c). The direct compression of a powder blend represents a straightforward, cost-efficient, reliable and high-throughput approach towards formulation, which represents an improvement in efficiency and reliability over alternative methods of production, for example in comparison to wet granulation followed by a subsequent tabletting. In one embodiment, the direct compression of the present invention further represents an inherent description of the structural features of the tablet composition.

In one embodiment of the invention, the pharmaceutical composition is characterized in that the composition further comprises a disintegrant, a glidant and/or a lubricant.

The pharmaceutical composition is characterized in that the water-soluble filler is a sugar.

In one embodiment of the invention, the pharmaceutical composition is characterized in that the water-soluble filler is mannitol.

In one embodiment of the invention, the pharmaceutical composition is characterized in that the water-soluble filler is lactose.

As described in EP0818454, known methods for manufacturing the compound bilastine can lead to the production of polymorphs 2 and/or 3 of bilastine. EP1505066 teaches subsequently the production of polymorph 1 from polymorphs 2 and/or 3 by having to conduct additional steps. In order to simplify the production of a pharmaceutical composition comprising bilastine, polymorph 2 was selected for further formulation studies, due to the absence of the requirement for further chemical processing that would otherwise be required for the production of polymorph 1.

As such, one object of the invention was to identify a simplified method of manufacture of a stable bilastine formulation with suitable disintegration and dissolution properties based on polymorph form 2.

When bilastine polymorph form 2 was formulated using excipients similar to that of the reference product, the dissolution profiles were incomplete compared to the reference product, as can be determined from the dissolution studies below. Despite showing acceptable disintegration, the dissolution of formulations using polymorph form 2 and those excipients of the commercial formulation was, surprisingly, significantly retarded when compared to commercial formulations using polymorph 1.

As a further surprise, the incorporation of a water-soluble filler, in particular a sugar, more preferably mannitol or lactose, lead to significantly improved dissolution properties, achieving complete release of the API, comparable to the commercial reference product.

The incorporation of a water-soluble filler, such as the sugars listed herein, is therefore a preferred embodiment of the formulations described herein employing polymorph form 2. It was entirely unexpected that a water-soluble filler would enable improved dissolution of the API, when the presence or absence of said water-soluble filler is non determinative for the disintegration of the tablet.

In one embodiment of the invention, the pharmaceutical composition is characterized in that the glidant is silicon

dioxide, preferably as colloidal anhydrous silica, magnesium aluminometasilicate and/or talc, preferably a combination of silicon dioxide and magnesium aluminometasilicate.

In a preferred embodiment, magnesium aluminometasilicate is employed, and has an unexpected stabilizing effect on the active component bilastine. In particular, the polymorphic form 2 is stabilized by magnesium aluminometasilicate in the compositions of the present invention and prevents formation of bilastine hydrates, as have been described for example in WO 2017/017301.

The presence of magnesium aluminometasilicate has been shown to convey multiple benefits upon the compositions of the present inventions, and shows an unexpected synergy with bilastine. The magnesium aluminometasilicate also functions as a glidant, providing effective glidant properties improving the flowability of the blend of components prior to formulation as a tablet. Furthermore, the magnesium aluminometasilicate does not impair the improved dissolution properties of bilastine achieved by employing a water-soluble filler, as discussed in detail below. In addition, the magnesium aluminometasilicate exerts a prevention of conversion from crystalline to hydrate forms of bilastine, thereby enhancing stability of the API.

Furthermore, the improved dissolution properties of tablets comprising the water-soluble fillers as disclosed herein could be maintained over longer periods of time, thereby generating a more stable set of dissolution properties, by incorporating a glidant in the tablet, preferably selected from silicon dioxide, preferably as colloidal anhydrous silica, magnesium aluminometasilicate and/or talc. It was entirely unexpected that the incorporation of a glidant, most preferably silicon dioxide and magnesium aluminometasilicate, would enable the maintenance of the beneficial dissolution properties enabled by the water-soluble fillers over long periods of time.

As such, the combination of a water-soluble filler with a glidant as described herein, based preferably on those particular examples and specific embodiments disclosed herein, would provide a synergistic effect, i. e. the improved dissolution of the API bilastine when present as polymorph 2, combined with the prolonged stability of these dissolution properties. A skilled person would have derived neither a suggestion nor motivation from the prior art that the excipients as described herein would lead to this combination of effects.

Furthermore, these two unexpected effects lead to a set of beneficial properties of the pharmaceutical formulation that is greater than the sum of these effects when considered alone. The presence of a glidant, preferably magnesium aluminometasilicate, talc and/or silica would not necessarily provide a beneficial effect alone. The combination of a glidant with a water-soluble filler, such as a sugar, provides an unexpected enhancement of the improved dissolution.

In one embodiment of the invention, the pharmaceutical composition comprises a crystalline form of bilastine, wherein the crystalline form has characteristic peaks at 6.53, 15.24, 15.86, and 18.07 $\pm$ 0.2 degrees 2-theta in a powder X-ray diffraction pattern, a water-soluble filler, a water-insoluble filler, a disintegrant, a glidant, and a lubricant, wherein the water-soluble filler is D-mannitol and the glidant is colloidal anhydrous silica or a combination of colloidal anhydrous silica and magnesium aluminometasilicate.

In one embodiment of the invention, the pharmaceutical composition is characterized in that the water-insoluble filler is microcrystalline cellulose. Microcrystalline cellulose is to be considered a preferred non-liming embodiment.

In one embodiment of the invention, the pharmaceutical composition is characterized in that the disintegrant is selected from sodium starch glycolate, low-substituted hydroxypropylcellulose, pregelatinized starch, or crospovidone, preferably sodium starch glycolate or crospovidone.

In one embodiment of the invention, the pharmaceutical composition is characterized in that the lubricant is a stearate, preferably magnesium stearate.

In one embodiment of the invention, the pharmaceutical composition is characterized in that the water-soluble filler is D-mannitol, the water-insoluble filler is microcrystalline cellulose, the disintegrant is sodium starch glycolate, the glidant is colloidal anhydrous silica or a combination of colloidal anhydrous silica and magnesium aluminometasilicate, and the lubricant is magnesium stearate.

In one embodiment of the invention, the pharmaceutical composition is characterized in that the tablet is a coated or uncoated immediate-release tablet, preferably uncoated.

In one embodiment of the invention, the pharmaceutical formulation is characterized in that

a. the bilastine polymorph form 2 is present in an amount of 10-20 wt%, preferably 12-18 wt%,

b. the water-soluble filler is present in an amount of 40-70 wt%, preferably 50-60 wt%,

c. the water-insoluble filler is present in an amount of 0-30 wt%, preferably 7-30 wt%, more preferably 20-30 wt%,

d. the disintegrant is present in an amount 0-5 wt%, 0.1-5 wt%, preferably 0.5-3 wt%,

e. the glidant is optionally present in an amount of 0-5 wt%, 0.1-5 wt%, preferably 0.5-3 wt%, and

f. the lubricant is present in an amount of 0-5 wt%, 0.1-5 wt%, preferably 0.5-3 wt%,

based on the total weight of all components of the tablet.

In a preferred embodiment the composition relates preferably to BIL/F1, BIL/F2, BIL/F3, BIL/F4, BIL/F5, BIL/F6, or BIL/F7, or alternative formulations based closely on these embodiments, as demonstrated below, in particular compositions with quantities of components falling within the ranges indicated by the "% range" parameters disclosed below.

The below embodiments may also be considered to encompass further embodiments of the invention in which the indicated amounts of the components are employed, but alternative water-soluble fillers, water insoluble fillers, disintegrants, glidants and/or lubricants, other than the specific components disclosed below, are used.

**Embodiments based on BIL/F1:**

| **S.No.** | **Ingredient** | **mg/tab** | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.07 | 15.80% | 10-20 |
| 2 | Lactose Monohydrate (Tablettose® 100) | Water-soluble filler | 72.17 | 56.83% | 40-70 |
| 3 | Cellulose, Microcrystalline *Ph. Eur.* (Avicel® PH 200) | Water-insoluble filler | 30.86 | 24.30% | 20-30 |
| 4 | Sodium Starch Glycolate *Ph. Eur.* (Explotab®) | Disintegrant | 1.00 | 0.79% | 0.1-5 |
| 5 | Silica, colloidal anhydrous *Ph. Eur.* (Aerosil® 200) | Glidant | 0.96 | 0.76% | 0.1-5 |
| | **Stage-B (Lubrication)** | | | | |
| 1 | Magnesium Stearate *Ph. Eur.* (Ferro) | Lubricant | 1.94 | 1.53% | 0.1-5 |
| | **Tablet Weight (mg):** | | **127.00** | 100.00% | 100.00% |

**Embodiments based on BIL/F2:**

| **S.No.** | **Ingredient** | **mg/tab** | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.10 | 16.08% | 10-20 |
| 2 | Mannitol *Ph. Eur.* (Pearlitol® 200 SD) | Water-soluble filler | 72.00 | 57.60% | 40-70 |
| 3 | Cellulose, Microcrystalline *Ph. Eur.* (Avicel® PH 200) | Water-insoluble filler | 29.60 | 23.68% | 20-30 |
| 4 | Sodium Starch Glycolate *Ph. Eur.* (Explotab®) | Disintegrant | 1.00 | 0.80% | 0.1-5 |
| 5 | Silica, colloidal anhydrous *Ph. Eur.* (Aerosil® 200) | Glidant | 1.00 | 0.80% | 0.1-5 |
| | **Stage-B (Lubrication)** | | | | |
| 1 | Magnesium Stearate *Ph. Eur.* (Ferro) | Lubricant | 1.30 | 1.04% | 0.1-5 |
| | **Tablet Weight (mg):** | | **125.00** | 100.00% | 100.00% |

**Embodiments based on BIL/F3:**

| S.No. | Ingredient | mg/tab | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.10 | 16.08% | 10-20 |
| 2 | Mannitol *Ph. Eur.* (Pearlitol® 200 SD) | Water-soluble filler | 99.40 | 79.52% | 70-90 |
| 3 | Crospovidone *Ph. Eur.* (Polyplasdone® XL-10) | Disintegrant | 2.50 | 2.00% | 1-5 |
| 5 | Silica, colloidal anhydrous *Ph. Eur.* (Aerosil® 200) | Glidant | 1.00 | 0.80% | 0.1-5 |
| | **Stage-B (Lubrication)** | | | | |
| 1 | Magnesium Stearate *Ph. Eur.* (Ferro) | Lubricant | 2.00 | 1.60% | 0.1-5 |
| | **Tablet Weight (mg):** | | **125.00** | 100.00% | 100.00% |

**Embodiments based on BIL/F4:**

| S.No. | Ingredient | mg/tab | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.10 | 16.08% | 10-20 |
| 2 | Mannitol *Ph. Eur.* (Pearlitol® 200 SD) | Water-soluble filler | 80.00 | 64.00% | 50-80 |
| 3 | Cellulose, Microcrystalline *Ph. Eur.* (Avicel® PH 200) | Water-insoluble filler | 16.90 | 13.52% | 7-20 |
| 4 | Crospovidone *Ph. Eur.* (Polyplasdone® XL-10) | Disintegrant | 6.00 | 4.80% | 1-15 |
| 5 | Silica, colloidal anhydrous *Ph. Eur.* (Aerosil® 200) | Glidant | 1.00 | 0.80% | 0.1-5 |
| | **Stage-B (Lubrication)** | | | | |
| 1 | Magnesium Stearate *Ph. Eur.* (Ferro) | Lubricant | 1.00 | 0.80% | 0.1-5 |
| | **Tablet Weight (mg):** | | **125.00** | 100.00% | 100.00% |

**Embodiments based on BIL/F5:**

| S.No. | Ingredient | mg/tab | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.15 | 16.12% | 10-20 |
| 2 | Mannitol *Ph. Eur.* (Pearlitol® 200 SD) | Water-soluble filler | 73.00 | 58.40% | 40-70 |
| 3 | Cellulose, Microcrystalline *Ph. Eur.* (Avicel® PH 200) | Water-insoluble filler | 28.60 | 22.88% | 15-30 |
| 4 | Crospovidone *Ph. Eur.* (Polyplasdone® XL-10) | Disintegrant | 0.50 | 0.40% | 0.1-5 |
| 5 | Silica, colloidal anhydrous *Ph. Eur.* (Aerosil® 200) | Glidant | 1.00 | 0.80% | 0.1-5 |

(continued)

| | | | | | |
|---|---|---|---|---|---|
| | **Stage-B (Lubrication)** | | | | |
| 1 | Magnesium Stearate *Ph. Eur.* (Ferro) | Lubricant | 1.75 | 1.40% | 0.1-5 |
| | **Tablet Weight (mg):** | | **125.00** | 100.00% | 100.00% |

**Embodiments based on BIL/F6:**

| **S.No.** | **Ingredient** | **mg/tab** | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.05 | 16.01 % | 10-20 |
| 2 | Mannitol *Ph. Eur.* (Pearlitol® 200 SD) | Water-soluble filler | 73.00 | 58.31% | 40-70 |
| 3 | Cellulose, Microcrystalline *Ph. Eur.* (Avicel® PH 200) | Water-insoluble filler | 26.20 | 20.93% | 10-30 |
| 4 | Crospovidone *Ph. Eur.* (Polyplasdone® XL-10) | Disintegrant | 0.50 | 0.40% | 0.1-5 |
| 5 | Silica, colloidal anhydrous *Ph. Eur.* (Aerosil® 200) | Glidant | 1.00 | 0.80% | 0.1-5 |
| 6 | Talc *Ph. Eur.* (Imerys) | Glidant | 2.50 | 2.00% | 0.1-5 |
| | **Stage-B (Lubrication)** | | | | |
| 1 | Magnesium Stearate *Ph. Eur.* (Ferro) | Lubricant | 1.94 | 1.55% | 0.1-5 |
| | **Tablet Weight (mg):** | | **125.00** | 100.00% | 100.00% |

**Embodiments based on BIL/F7:**

| **S.No.** | **Ingredient** | **mg/tab** | | | |
|---|---|---|---|---|---|
| | **Stage-A (Sifting & Blending)** | **Function** | **mg/tab** | **wt%** | **Preferred % range** |
| 1 | Bilastine *(In house)* | API | 20.05 | 16.04% | 10-20 |
| 2 | Mannitol *Ph. Eur.* (Pearlitol® 200 SD) | Water-soluble filler | 73.00 | 58.40% | 40-70 |
| 3 | Cellulose, Microcrystalline *Ph. Eur.* (Avicel® PH 200) | Water-insoluble filler | 24.80 | 19.84% | 10-30 |
| 4 | Crospovidone *Ph. Eur.* (Polyplasdone® XL-10) | Disintegrant | 1.90 | 1.52% | 0.1-5 |
| 5 | Silica, colloidal anhydrous *Ph. Eur.* (Aerosil® 200) | Glidant | 1.00 | 0.80% | 0.1-5 |
| 6 | Talc *Ph. Eur.* (Luzenac) | Glidant | 2.50 | 2.00% | 0.1-5 |
| | **Stage-B (Lubrication)** | | | | |
| 1 | Magnesium Stearate *Ph. Eur.* (Valtris) | Lubricant | 1.75 | 1.40% | 0.1-5 |
| | **Tablet Weight (mg):** | | **125.00** | 100.00% | 100.00% |

**Embodiments based on BIL/F8:**

| S.No. | Ingredient | mg/tab | | | |
|---|---|---|---|---|---|
| | **Stage-A (Sifting & Blending)** | **Function** | **mg/tab** | **wt%** | **Preferred % range** |
| 1 | Bilastine *(In house)* | API | 20,149 | 16,12% | 10-20 |
| 2 | Mannitol *Ph. Eur.* (Pearlitol® 200 SD) | Water-soluble filler | 73,000 | 58,40% | 40-70 |
| 3 | Cellulose, Microcrystalline *Ph. Eur.* (Avicel® PH 200) | Water-insoluble filler | 24,701 | 19,76% | 10-30 |
| 4 | Crospovidone *Ph. Eur.* (Polyplasdone® XL-10) | Disintegrant | 1,900 | 1,52% | 0.1-5 |
| 5 | Silica, colloidal anhydrous *Ph. Eur.* (Aerosil® 200) | Glidant | 1,000 | 0,80% | 0.1-5 |
| 6 | Magnesium Aluminometasilicate Ph. Eur. (Neusilin® US2) | Glidant | 2,500 | 2,00% | 0.1-5 |
| | **Stage-B (Lubrication)** | | | | |
| 1 | Magnesium Stearate *Ph. Eur.* (Valtris) | Lubricant | 1,750 | 1,40% | 0.1-5 |
| | **Tablet Weight (mg):** | | **125.00** | 100.00% | 100.00% |

**Embodiments based on BIL/F9:**

| S.No. | Ingredient | mg/tab | | | |
|---|---|---|---|---|---|
| | **Stage-A (Blending and granulating)** | **Function** | **mg/tab** | **wt%** | **Preferred % range** |
| 1 | Bilastine (In house) | API | 20,09 | 16,07% | 10-20 |
| 2 | Mannitol Ph. Eur. (Pearlitol® 200 SD) | Water-soluble filler | 73,00 | 58,40% | 40-70 |
| 3 | Cellulose, Microcrystalline Ph. Eur. (Avicel® PH 200) | Water-insoluble filler | 24,91 | 19,93% | 10-30 |
| 4 | Sodium Starch Glycolate Ph. Eur. (Explotab®) | Disintegrant | 1,50 | 1,20% | 0.1-5 |
| 5 | Silica, colloidal anhydrous Ph. Eur. (Aerosil® 200 Pharma) | Glidant | 0,50 | 0,40% | 0.1-5 |
| 6 | Magnesium Aluminometasilicate Ph. Eur. (Neusilin® US2) | Glidant | 2,50 | 2,00% | 0.1-5 |
| 7 | Magnesium Stearate Ph. Eur. (Valtris) | Lubricant | 1,00 | 0,80% | 0.1-5 |
| | **Stage-B (Lubrication)** | | | | |
| 1 | Silica, colloidal anhydrous Ph. Eur. (Aerosil® 200 Pharma) | Glidant | 0,50 | 0,40% | 0.1-5 |
| 2 | Magnesium Stearate Ph. Eur. (Valtris) | Lubricant | 1,00 | 0,80% | 0.1-5 |
| | **Tablet Weight (mg):** | | **125.00** | 100.00% | 100.00% |

In the potential embodiments based on BIL/F9, the total amount of glidant and/or lubricant, whether added in the stage A and/or stage B, is preferably 0.1-5% based on the total weight of all components of the tablet.

The invention further relates to a pharmaceutical composition as described herein for use as a medicament in the treatment of allergic rhino-conjunctivitis and/or urticaria. As such, the invention relates to the use of a pharmaceutical composition as described herein in the manufacture of a medicament for the treatment of allergic rhino-conjunctivitis

and/or urticaria. The invention further relates to a method of treatment of allergic rhino-conjunctivitis and/or urticaria, comprising administering to a patient in need thereof a pharmaceutical composition in accordance with the composition described herein, preferably a composition comprising a therapeutically relevant or efficacious amount of bilastine.

The invention further relates to a method of preparing a pharmaceutical composition in the form of a tablet, comprising dry granulation of a blend and compression of the granules to a tablet, wherein the blend comprises a) a crystalline form of bilastine, wherein the crystalline form has characteristic peaks at 6.53, 15.24, 15.86, and 18.07 ± 0.2 degrees 2-theta in a powder X-ray diffraction pattern, b) a water-soluble filler, wherein the water-soluble filler is a sugar, and optionally c) a water-insoluble filler.

In a preferred embodiment, the method comprises blending of the components a), b) and optionally c), roller compaction and granulation of the blend, milling the granules, lubricating the granules and subsequent compression of the granules to a tablet.

A dry granulation method is surprisingly advantageous with respect to minimizing the impact of API variability on processing.

The invention further relates to a method of preparing a pharmaceutical composition in the form of a tablet, comprising direct compression of a powder blend, wherein said powder blend comprises a) a crystalline form of bilastine, wherein the crystalline form has characteristic peaks at 6.53, 15.24, 15.86, and 18.07 ± 0.2 degrees 2-theta in a powder X-ray diffraction pattern, b) a water-soluble filler, wherein the water-soluble filler is a sugar, and optionally c) a water-insoluble filler.

As shown in more detail below, in the form of examples, the method of the present invention enables a straightforward, cost-efficient, reliable and high-throughput approach towards formulation of bilastine, which represents an improvement in efficiency and reliability over alternative methods of production. The method is capable of producing stable bilastine formulations, with excellent content uniformity within the specification limits according to European Pharmacopeia 2.9.40 independent of changes in the blending parameters (pre-lubrication time, lubrication time, and blender speed).

In one embodiment of the invention, the method for preparing the pharmaceutical composition is characterized in that the method comprises:

- Dispensing the blend components, preferably comprising a) a crystalline form of bilastine, wherein the crystalline form has characteristic peaks at 6.53, 15.24, 15.86, and 18.07 ± 0.2 degrees 2-theta in a powder X-ray diffraction pattern, b) a water-soluble filler, wherein the water-soluble filler is a sugar, and optionally c) a water-insoluble filler;
- Sifting bilastine and the filler(s) as a blend through a suitable mesh;
- Optionally sifting the disintegrant, glidant(s) and lubricant separately through a suitable mesh /this step may also be combined with the step above, enabling a single sifting step);
- Loading the materials above (preferably with the exception of the lubricant or with only a portion of the lubricant) in a blender and mixing for sufficient time to mix the components (pre-lubrication blending);
- Dry granulation of the blend, preferably using roller compaction;
- Optionally milling the granules in order to achieve the desired granule size;
- Optionally adding the lubricant (if not already incorporated, or adding the remaining portion of the lubricant) and continue the blending for sufficient time to mix the granules with the lubricant (lubrication blending);
- Compressing the granules into tablets using the (lubricated) granules of the previous step.

In one embodiment of the invention, the method for preparing the pharmaceutical composition is characterized in that the method comprises:

- Dispensing the blend components, preferably comprising a) a crystalline form of bilastine, wherein the crystalline form has characteristic peaks at 6.53, 15.24, 15.86, and 18.07 ± 0.2 degrees 2-theta in a powder X-ray diffraction pattern, b) a water-soluble filler, wherein the water-soluble filler is a sugar, and optionally c) a water-insoluble filler;
- Sifting bilastine and the filler(s) as a blend through a suitable mesh;
- Optionally sifting the disintegrant, glidant(s) and lubricant separately through a suitable mesh;

- Loading the materials above, preferably with the exception of the lubricant, in a blender and mixing for sufficient time to mix the components (pre-lubrication blending);

- Optionally adding the lubricant (if not already incorporated) and continue the blending for sufficient time to mix the components (lubrication blending);

- Compressing the tablets using the blend of the previous step.

In one embodiment of the invention, the method for preparing the pharmaceutical composition is characterized in that the pre-lubrication blending is carried out for 1-60 minutes, preferably 5-30 minutes, more preferably 8-25 minutes, in particular 10, 15 or 20 minutes.

In one embodiment of the invention, the method for preparing the pharmaceutical composition is characterized in that the lubrication blending is carried out for 0-30 minutes, preferably 1-10 minutes, more preferably 2-8 minutes, in particular 3, 5 or 7 minutes.

In one embodiment of the invention, the method for preparing the pharmaceutical composition is characterized in that the blending is conducted in a blender with 5-100 rpm, preferably 5-40 rpm, in particular 10, 20 or 30 rpm blending speed.

## DETAILED DESCRIPTION OF THE INVENTION

"Bilastine" or (name 2-[4-(2-(4-(1-(2-ethoxyethyl)-1H-benzimidazol-2-yl)piperidin-1-yl)ethyl)phenyl]-2-methylpropionic acid), recorded under CAS number 202189-78-4 is known as a second generation antihistamine drug for the treatment of allergic rhinoconjunctivitis and urticaria (hives). It exerts its effect as a selective histamine H1 receptor antagonist, and has an effectiveness similar to cetirizine, fexofenadine and desloratadine. Bilastine is approved in the European Union for the symptomatic treatment of allergic rhinoconjunctivitis and urticaria.

The term "bilastine" as used herein according to the present invention includes bilastine in the form of free base, a pharmaceutically acceptable salt thereof, amorphous bilastine, crystalline bilastine, preferably selected from the polymorph forms described herein, any isomer, derivative, hydrate, solvate or prodrug or a combination thereof.

Bilastine or polymorphs thereof may also be prepared as a pharmaceutical salt. Examples of pharmaceutical acceptable salts of bilastine which can be contained as an active ingredient in a solid oral dosage form include acid addition salt formed with mineral acids such as hydrochloric acid, hydrobromic acid, hydroiodic acid, sulfuric acid, nitric acid, phosphoric acid and the like; acid addition salts formed with organic acids such as acetic acid, propionic acid, butyric acid, oxalic acid, citric acid, succinic acid, tartaric acid, fumaric acid, malic acid, lactic acid, adipic acid, benzoic acid, salicylic acid, methanesulfonic acid, benzenesulfonic acid, p-toluenesulfonic acid, glutamic acid, aspartic acid and the like. Examples of solvate include solvates with water, ethyl alcohol or the like.

The polymorph forms 1, 2 and 3 of bilastine are known from the prior art, for example in the descriptions of EP1505066, WO 2014/026657 and WO 2017/017301.

All references to the "polymorph I form", "polymorph 1 form", "polymorph 1", "polymorph I", "form 1" or "form I", "crystalline form 1" or "crystalline form I" or the like may be used interchangeably.

Form 1, described in example 12 of WO 2014026657 relates to a crystalline form with the following characteristics:

XRPD ([°2Th.] (rel. int %)): 3.64 (4.4), 10.57 (23.3), 11.27 (78.1), 12.47 (38.8), 14.08 (26.9), 15.07 (38.4), 15.50 (16.5), 16.27 (43.6), 17.16 (100.0), 18.89 (71.8), 19.73 (74.0), 21.13 (33.9), 22.17 (18.1), 22.71 (26.9), 23.34 (10.3), 24.88 (18.6), 25.82 (9.2), 26.58 (1 1.5), 28.43 (9.7), 29.16 (8.8), 30.92 (4.6), 34.38 (9.5), 37.01 (5.4).

The figure 1 of WO2017/017301 shows the XRD pattern of form 1 with essentially the same peak positions as the pattern of form 1 disclosed in the figure 7 of WO 2014/026657.

The inventors have also analysed bilastine form 1 and find the characteristic diffraction peaks to be present at 10.57, 11.27, 12.44, 14.08, 15.07, 15.50, 16.27, and 17.16 ± 0.2° 2Theta.

For the monitoring of bilastine form 1 presence in the tablets, the most suitable peaks are the characteristic and non-interfering reflexes at 12.47 and/or 14.08 ± 0.2° 2Theta.

In embodiments of the present invention, the polymorph 2 form is preferred. All references to the "polymorph II form", "polymorph 2 form", "polymorph 2", "polymorph II", "form 2" or "form II", "crystalline form 2" or "crystalline form II" or the like may be used interchangeably.

Form 2, described in example 12 of WO 2014026657 relates to a crystalline form with the following characteristics:

XRPD ([°2Th.] (rel. int %)): 6.53 (100.0), 9.43 (30.8), 11.04 (22.8), 13.39 (6.2), 15.24 (32.2), 15.86 (86.1), 18.07 (29.9), 18.39 (36.2), 18.94 (8.3), 20.19 (16.0), 20.66 (19.0), 21.70 (17.1), 22.17 (15.6), 23.70 (5.7), 26.59 (4.9), 28.03 (3.6), 28.33 (3.6), 29.70 (4.3).

The inventors have also analysed bilastine form 2 and find the characteristic diffraction peaks to be present at 6.53°, 15.24°, 15.86°, and 18.07° (plus-minus 0.2°) 2Theta.

In some embodiments, two additional reflexes (characteristic diffraction peaks) at 9.43° and 11.04°, which are also characteristic and well visible in the XRPD analyses conducted despite signals from placebo (mainly from Mannitol, which is highly crystalline), may be employed for a definition of the crystalline form 2.

Preferably, characteristic reflexes for Form 2 are 6.53°, 9.43°, 11.04, 15.24°, 15.86°, and 18.07° ± 0.2° 2Theta. As such, no characteristic peaks are observed for form 1, for example at 12.47 or 14.08 ± 0.2° 2Theta.

The term "essentially the same" with reference to PXRD means that variabilities in peak positions and relative intensities of the peaks are to be taken into account. For example, a typical precision of the 2-Theta values is in the range of ± 0.2° 2-Theta. Thus, for example a diffraction peak that usually appears at 15.7° 2-Theta for example can appear between 15.5° and 15.9 ° 2-Theta on most X-ray diffractometers under standard conditions. With respect to the relative intensities and the characteristic peaks of the powder X-ray diffraction patterns mentioned above, the provided values of relative intensity are not intended as limiting for the identification of the characteristic peaks mentioned. As is known to a skilled person, the relative peak intensities will show inter-apparatus variability, batch-to-batch variability, as well as variability due to degree of crystallinity, preferred orientation, sample preparation, and as such are provided as an indication and as qualitative measure only, but not a limiting definition, of the intensities of the peaks in the powder X-ray diffraction patterns. The term "characterizing peak" in the context of defining the present invention is therefore not limited to the respective relative intensities provided above, and any one or more of the respective peaks may be determined as a characterizing peak for any given form of bilastine. Preferably at least 1, 2, 3 or 4 peaks are used to characterize a bilastine polymorphic form, in other embodiments, at least 5, 6, 7, 8 9 or 10 peaks may be employed.

The term "active ingredient" or "API" herein refers to a pharmaceutically active molecule (e.g. bilastine) as well as its pharmaceutically acceptable and therapeutically active salts, esters, amides, prodrugs, metabolites, enantiomers, polymorphs, analogs, etc. that induce a desired pharmacological or physiological effect. Terms like "active", "active agent", "active substance" may be used synonymously for "active ingredient".

The term "effective amount" or "therapeutically effective amount" used interchangeably, is defined to mean the amount or quantity of the active drug (e.g. bilastine), which is sufficient to elicit an appreciable biological response when administered to the patient. It will be appreciated that the precise therapeutic dose will depend on the age and condition of the patient, nature of the condition to be treated and will be at the ultimate discretion of the attendant physician.

The term "excipient" means a pharmacologically inactive component such as a diluent, disintegrant, carrier, and the like, of a pharmaceutical product. The excipients that are useful in preparing a pharmaceutical composition are generally safe, non-toxic and are acceptable for veterinary as well as human pharmaceutical use. Reference to an excipient includes both one excipient and more than one excipient.

The excipients are described herein in some embodiments according to "wt%", or "percentage by weight". The %wt values recite herein preferably relate to the percentage of material by weight present in the tablet, or in the powder blend prior to compression.

According to the invention, a water-soluble filler may be used, i.e. as a bulking agent. Various useful water-soluble fillers include but are not limited to sugars, such as mannitol, lactose, sorbitol, xylitol, and the like, and mixtures thereof; more preferably selected from mannitol and lactose.

According to the invention, a water-insoluble filler may be used. Various useful water-insoluble fillers include but are not limited to starch, powdered cellulose, microcrystalline cellulose (MCC), calcium phosphate and the like, or combinations thereof.

According to the present invention, one or more lubricants can be used. Useful lubricants include but are not limited to stearates, such as magnesium stearate, or sodium stearyl fumarate.

According to the present invention, one or more glidants can be used. Useful glidants include but are not limited to silica, in various forms, such as colloidal silicon dioxide, magnesium aluminometasilicate (also known as Neusilin), magnesium silicate, magnesium trisilicate, talc, and other forms of silicon dioxide, such as aggregated silicates and hydrated silica.

Magnesium aluminometasilicate (also known as Silodrate or Simaldrate) typically occurs as a white powder, grain, or granule. It is commonly known according to the following formula $Al_2O_2.2Mg.3O_3Si$ with a MW of 362.821 g/mol. Magnesium aluminometasilicate may also be identified with the following formula $Al_2H_2Mg_2O_{12}Si_3$ with a MW of 380.832 g/mol. Magnesium aluminometasilicate may also be identified with the following formula $Al_2O_3.MgO.1.7SiO_2.xH_2O$ (CAS number 12511-31-8). In a preferred embodiment, the magnesium aluminometasilicate is employed as Neusilin®, which is a fine granule of magnesium aluminometasilicate.

According to the present invention, a disintegrant is typically an agent used in the preparation of solid pharmaceutical formulations which causes them to disintegrate and release their medicinal substances on contact with moisture. Disintegrants include but are not limited to hydroxypropyl cellulose (L-HPC), pregelatinized starch (PGS), crospovidone, croscarmellose sodium, sodium starch glycolate and the like.

The composition may also comprise other excipients, such as surfactants and/or binders, as may be desired.

A "tablet" as used herein is considered a solid unit dosage form of a medicament comprising one or more excipients.

The most commonly used pharmaceutical solid dosage forms today include granules, pellets, tablets and capsules. Tablets are solid pharmaceutical dosage forms containing drug substances with one or more excipients prepared by either compression or molding methods. The basic art of tableting by three well known methods includes direct compression, wet granulation and dry granulation. According to the present invention dry granulation and direct compression are preferred. Dry granulation may typically be employed if the materials have sufficient inherent binding or cohesive properties to form granules. Dry granulation refers typically to the process of granulating without the use of liquids. Two dry granulation methods are primarily used in the pharmaceutical industry, namely slugging and roller compaction. In a roller compactor material particles are consolidated and densified by passing the material between two high pressure rollers. The densified material from a roller compactor is then reduced to a uniform granule size by milling. Roll compaction/dry granulation (RCDG) is a method of choice for processing of physically or chemically moisture sensitive drugs, as no liquid binder is required in the granulation, and is a preferred method of the present invention.

In the dry granulation method of tablet production, dry ingredients are thoroughly mixed to a blend, the blend is granulated, for example by roller compaction and granulation and optionally milling, and the granules are then compressed into tablets. This eliminates the drying steps associated with the wet granulation method. It also reduces the higher costs involved in wet granulation including increased equipment, labor, time, process validation and energy expenditure. As a result, dry granulation is both efficient and economical, well suited to the production of high quality tablets, which exhibit hardness, low friability and excellent dissolution rates. As an added benefit, dry granulation can improve the physical and chemical stability of tablets as compared to wet granulation.

In the direct compression method of tablet production, dry ingredients are thoroughly mixed to a powder blend and then compressed into tablets. This eliminates the drying steps associated with the wet granulation method. It also reduces the higher costs involved in wet granulation including increased equipment, labor, time, process validation and energy expenditure. As a result, direct compression is both efficient and economical, well suited to the production of high quality tablets, which exhibit hardness, low friability and excellent dissolution rates. As an added benefit, direct compression can improve the physical and chemical stability of tablets as compared to wet granulation.

According to the invention, a powder or powder blend is compressed, which is a substantially dry solid, composed of preferably a large number of fine particles that may flow when shaken or tilted. The powder of the present invention is preferably distinguishable from granules, which are typically prepared by wet or dry granulation, and are typically larger than the particles of the powder of the invention. According to one embodiment of the invention, the powder blend is directly compressed.

Methods for compression of tablets are known to a skilled person and may be elected appropriately without undue effort. For example, after mixing of ingredients (in case of direct compression), the powder blend may be directly compressed to obtain a tablet. Methods for compression of granules into tablets are also known to a skilled person, wherein granules are subsequently compressed into tablets. The compression is preferably carried out by either by single punch machine (stamping press) or by multi station machine (rotary press). The tablet press is a high-speed mechanical device. It 'squeezes' the ingredients into the required tablet shape with extreme precision. It can make the tablet in many shapes, although they are usually round or oval. Also, it can press the name of the manufacturer or the product into the top of the tablet. Allergic rhinoconjunctivitis, or Rhinitis, or Allergic rhinitis, or hay fever, is typically caused by an inflammation in the nose which occurs when the immune system overreacts to allergens in the air. Rhinitis is characterized by nasal congestion, runny nose, post-nasal drip, sneezing, red eyes and/or itching of the nose or eyes. Allergic rhinitis is typically associated with the presence of antibodies (IgE) which recognize certain allergens. When these antibodies are exposed to the allergen, they bind the allergen and this leads to an inflammatory reaction. This reaction is characterized by the liberation of several inflammatory mediators, which in turn leads to the signs and symptoms typical of allergic rhinitis.

Urticaria, or hives, is a vascular reaction of the skin marked by the transient appearance of smooth plaques (wheals). Individual lesions typically resolve without scarring in several hours. Most cases of urticaria are self-limited and of short duration; the eruption rarely lasts more than several days, it but may be recurrent over weeks. Chronic urticaria is also treatable using the API as described herein. A medical practitioners is capable of electing a suitable dose of the tablet described herein for treatment of allergic rhinoconjunctivitis or urticaria, depending on the age and size of the patient, and severity of disease, or other relevant factors.

## FIGURES

The invention is demonstrated by way of the figures disclosed herein. The figures provide support for a detailed description of potentially preferred, non-limiting embodiments of the invention.

Figure 1 is showing the powder XRD of Form 1 Bilastine API.

Figure 2 is showing the powder XRD of Form 2 Bilastine API.

Figure 3 is showing the powder XRD of tablets BIL/F5 (Initial).

Figure 4 is showing the powder XRD of tablets BIL/F5 (40°C/75%RH, 1 month in Alu-Alu blister).

Figure 5 is showing the powder XRD of tablets BIL/F7 (Initial).

Figure 6 is showing the powder XRD of tablets BIL/F7 (40°C/75%RH, 1 month in Alu-Alu blister).

Figure 7 is showing the powder XRD of tablets BIL/F8 (70°C/75%RH 2 Days open exposure).

Figure 8 is showing the powder XRD of tablets BIL/F8 (50°C/75%RH 14 Days open exposure).

Figure 9 is showing the powder XRD of tablets BIL/F9 (Initial).

Figure 10 is showing the powder XRD of tablets BIL/F9 (40°C/75%RH, 1 month in Alu-Alu blister).

Figure 11 is showing the powder XRD of tablets BIL/F9 (70°C/75%RH 2 Days open exposure).

## EXAMPLES

The invention is demonstrated by way of the examples disclosed herein. The examples provide technical support for a detailed description of potentially preferred, non-limiting embodiments of the invention.

### Part A - Comparative examples

**Comparative example 1 (BIL/C1):**

| S.No. | Ingredient | mg/tab | |
|---|---|---|---|
| | **Stage-A (Sifting & Blending)** | **Function** | **mg/tab** |
| 1 | Bilastine *(In house)* | API | 20.139 |
| 2 | Cellulose, Microcrystalline *Ph. Eur.* (Avicel® PH 200) | Water-insoluble filler | 102.861 |
| 3 | Sodium Starch Glycolate *Ph. Eur.* (Explotab®) | Disintegrant | 1.000 |
| 4 | Silica, colloidal anhydrous *Ph. Eur.*(Aerosil® 200) | Glidant | 0.960 |
| | **Stage-B (Lubrication)** | | |
| 1 | Magnesium Stearate *Ph.* Eur.(Ferro) | Lubricant | 1.940 |
| | **Tablet Weight (mg):** | | **126.900** |

**Manufacturing procedure (direct compression process):**

1.0 Dispense the materials according to the above indicated amounts.

2.0 Sift Bilastine and Microcrystalline Cellulose through suitable mesh.

3.0 Sift Sodium Starch Glycolate, Colloidal Anhydrous Silica, and Magnesium Stearate separately through suitable mesh.

4.0 Load the materials from steps 2.0 and 3.0 except Magnesium Stearate in blender and mix for sufficient time.

5.0 Add Magnesium Stearate from Step 2.0 to 4.0 and continue the blending for sufficient time.

6.0 Compress the tablets using blend from step 5.0.

**Comparative example 2 (BIL/C2):**

| S.No. | Ingredient | mg/tab | |
|---|---|---|---|
| | **Stage-A (Blending&Compaction)** | **Function** | **mg/tab** |
| 1 | Bilastine *(In house)* | API | 20.072 |
| 2 | Cellulose, Microcrystalline *Ph. Eur.* (Avicel® PH 200) | Water-insoluble filler | 101.968 |
| 3 | Sodium Starch Glycolate *Ph. Eur.* (Explotab®) | Disintegrant | 1.000 |
| 4 | Magnesium Stearate *Ph. Eur.* (Ferro) | Lubricant | 0.500 |
| | **Stage-B (Lubrication)** | | |
| 1 | Silica, colloidal anhydrous *Ph. Eur.* (Aerosil® 200) | Glidant | 0.960 |
| 2 | Magnesium Stearate *Ph. Eur.* (Ferro) | Lubricant | 1.940 |
| | **Tablet Weight (mg):** | | **126.440** |

**Manufacturing procedure (dry granulation process):**

1.0 Dispense the materials according to the above indicated amounts.

2.0 Sift Bilastine and Microcrystalline Cellulose through suitable mesh.

3.0 Sift Sodium Starch Glycolate, Colloidal Anhydrous Silica, and Magnesium Stearate separately through suitable mesh.

4.0 Load Sodium Starch Glycolate, Magnesium Stearate and the materials from the above step 2.0 in blender and mix for sufficient time.

5.0 The blend from step 4.0 is slugged on compression machine to prepare the compacts.

6.0 The compacts are milled using Quadro® *Comil*® to get suitable sized granules.

7.0 Load the granules from Step 6.0 into the blender. Add second portion of sifted Magnesium Stearate from Step 3.0 into the blender and continue the blending for sufficient time.

8.0 Compress the tablets using blend from step 7.0.

**Part B-1**

**General procedure for the preparation of inventive examples below employing a direct compression process:**

1.0 Dispense the materials according to the indicated amounts.

2.0 Sift Bilastine and Filler(s) through suitable mesh.

3.0 Sift Disintegrant, Glidant(s) and Lubricant separately through suitable mesh.

4.0 Load the materials from steps 2.0 and 3.0 except Lubricant in blender and mix for sufficient time.

5.0 Add Lubricant from Step 2.0 to 4.0 and continue the blending for sufficient time.

6.0 Compress the tablets using blend from step 5.0.

The following compositions were obtained using the above process:

## Inventive examples

**Example BIL/F1:**

| S.No. | Ingredient | mg/tab | |
|---|---|---|---|
| | **Stage-A (Sifting & Blending)** | **Function** | **mg/tab** |
| 1 | Bilastine *(In house)* | API | 20.072 |
| 2 | Lactose Monohydrate (Tablettose® 100) | Water-soluble filler | 72.170 |
| 3 | Cellulose, Microcrystalline *Ph. Eur.* (Avicel® PH 200) | Water-insoluble filler | 30.858 |
| 4 | Sodium Starch Glycolate *Ph. Eur.* (Explotab®) | Disintegrant | 1.000 |
| 5 | Silica, colloidal anhydrous *Ph. Eur.* (Aerosil® 200) | Glidant | 0.960 |
| | **Stage-B (Lubrication)** | | |
| 1 | Magnesium Stearate *Ph. Eur.* (Ferro) | Lubricant | 1.940 |
| | **Tablet Weight (mg):** | | **127.000** |

**Example BIL/F2:**

| S.No. | Ingredient | mg/tab | |
|---|---|---|---|
| | **Stage-A (Sifting & Blending)** | **Function** | **mg/tab** |
| 1 | Bilastine *(In house)* | API | 20.098 |
| 2 | Mannitol *Ph. Eur.* (Pearlitol® 200 SD) | Water-soluble filler | 72.000 |
| 3 | Cellulose, Microcrystalline *Ph. Eur.* (Avicel® PH 200) | Water-insoluble filler | 29.602 |
| 4 | Sodium Starch Glycolate *Ph. Eur.* (Explotab®) | Disintegrant | 1.000 |
| 5 | Silica, colloidal anhydrous *Ph. Eur.* (Aerosil® 200) | Glidant | 1.000 |
| | **Stage-B (Lubrication)** | | |
| 1 | Magnesium Stearate *Ph. Eur.* (Ferro) | Lubricant | 1.300 |
| | **Tablet Weight (mg):** | | **125.000** |

**Example BIL/F3:**

| S.No. | Ingredient | mg/tab | |
|---|---|---|---|
| | **Stage-A (Sifting & Blending)** | **Function** | **mg/tab** |
| 1 | Bilastine *(In house)* | API | 20.098 |
| 2 | Mannitol *Ph. Eur.* (Pearlitol® 200 SD) | Water-soluble filler | 99.402 |
| 3 | Crospovidone *Ph. Eur.* (Polyplasdone® XL-10) | Disintegrant | 2.500 |
| 5 | Silica, colloidal anhydrous *Ph. Eur.* (Aerosil® 200) | Glidant | 1.000 |

(continued)

| | | | |
|---|---|---|---|
| | **Stage-B (Lubrication)** | | |
| 1 | Magnesium Stearate *Ph. Eur.* (Ferro) | Lubricant | 2.000 |
| | **Tablet Weight (mg):** | | **125.000** |

**Example BIL/F4:**

| S.No. | Ingredient | mg/tab | |
|---|---|---|---|
| | **Stage-A (Sifting & Blending)** | **Function** | **mg/tab** |
| 1 | Bilastine *(In house)* | API | 20.098 |
| 2 | Mannitol *Ph. Eur.* (Pearlitol® 200 SD) | Water-soluble filler | 80.000 |
| 3 | Cellulose, Microcrystalline *Ph. Eur.* (Avicel® PH 200) | Water-insoluble filler | 16.902 |
| 4 | Crospovidone *Ph. Eur.* (Polyplasdone® XL-10) | Disintegrant | 6.000 |
| 5 | Silica, colloidal anhydrous *Ph. Eur.* (Aerosil® 200) | Glidant | 1.000 |
| | **Stage-B (Lubrication)** | | |
| 1 | Magnesium Stearate *Ph. Eur.* (Ferro) | Lubricant | 1.000 |
| | **Tablet Weight (mg):** | | **125.000** |

**Example BIL/F5:**

| S.No. | Ingredient | mg/tab | |
|---|---|---|---|
| | **Stage-A (Sifting & Blending)** | **Function** | **mg/tab** |
| 1 | Bilastine *(In house)* | API | 20.149 |
| 2 | Mannitol *Ph. Eur.* (Pearlitol® 200 SD) | Water-soluble filler | 73.000 |
| 3 | Cellulose, Microcrystalline *Ph. Eur.* (Avicel® PH 200) | Water-insoluble filler | 28.601 |
| 4 | Crospovidone *Ph. Eur.* (Polyplasdone® XL-10) | Disintegrant | 0.500 |
| 5 | Silica, colloidal anhydrous *Ph. Eur.* (Aerosil® 200) | Glidant | 1.000 |
| | **Stage-B (Lubrication)** | | |
| 1 | Magnesium Stearate *Ph. Eur.* (Ferro) | Lubricant | 1.750 |
| | **Tablet Weight (mg):** | | **125.000** |

**Example BIL/F6:**

| S.No. | Ingredient | mg/tab | |
|---|---|---|---|
| | **Stage-A (Sifting & Blending)** | **Function** | **mg/tab** |
| 1 | Bilastine *(In house)* | API | 20.046 |
| 2 | Mannitol *Ph. Eur.* (Pearlitol® 200 SD) | Water-soluble filler | 73.000 |
| 3 | Cellulose, Microcrystalline *Ph. Eur.* (Avicel® PH 200) | Water-insoluble filler | 26.204 |
| 4 | Crospovidone *Ph. Eur.* (Polyplasdone® XL-10) | Disintegrant | 0.500 |
| 5 | Silica, colloidal anhydrous *Ph. Eur.* (Aerosil® 200) | Glidant | 1.000 |
| 6 | Talc *Ph. Eur.* (Imerys) | Glidant | 2.500 |

(continued)

| | | | |
|---|---|---|---|
| | **Stage-B (Lubrication)** | | |
| 1 | Magnesium Stearate *Ph. Eur.* (Ferro) | Lubricant | 1.940 |
| | **Tablet Weight (mg):** | | **125.000** |

**Example BIL/F7:**

| S.No. | Ingredient | mg/tab | |
|---|---|---|---|
| | **Stage-A (Sifting & Blending)** | **Function** | **mg/tab** |
| 1 | Bilastine *(In house)* | API | 20.046 |
| 2 | Mannitol *Ph. Eur.* (Pearlitol® 200 SD) | Water-soluble filler | 73.000 |
| 3 | Cellulose, Microcrystalline *Ph. Eur.* (Avicel® PH 200) | Water-insoluble filler | 24.804 |
| 4 | Crospovidone *Ph. Eur.* (Polyplasdone® XL-10) | Disintegrant | 1.900 |
| 5 | Silica, colloidal anhydrous *Ph. Eur.* (Aerosil® 200) | Glidant | 1.000 |
| 6 | Talc *Ph. Eur.* (Luzenac) | Glidant | 2.500 |
| | **Stage-B (Lubrication)** | | |
| 1 | Magnesium Stearate *Ph. Eur.* (Valtris) | Lubricant | 1.750 |
| | **Tablet Weight (mg):** | | **125.000** |

**Example BIL/F8:**

| S.No. | Ingredient | mg/tab | |
|---|---|---|---|
| | **Stage-A (Sifting & Blending)** | **Function** | **mg/tab** |
| 1 | Bilastine *(In house)* | API | 20.149 |
| 2 | Mannitol *Ph. Eur.* (Pearlitol® 200 SD) | Water-soluble filler | 73.000 |
| 3 | Cellulose, Microcrystalline *Ph. Eur.* (Avicel® PH 200) | Water-insoluble filler | 24.701 |
| 4 | Crospovidone *Ph. Eur.* (Polyplasdone® XL-10) | Disintegrant | 1.900 |
| 5 | Silica, colloidal anhydrous *Ph. Eur.* (Aerosil® 200) | Glidant | 1.000 |
| 6 | Magnesium Aluminometasilicate *Ph. Eur.* (Neusilin® US2) | Glidant | 2.500 |
| | **Stage-B (Lubrication)** | | |
| 1 | Magnesium Stearate *Ph. Eur.* (Valtris) | Lubricant | 1.750 |
| | **Tablet Weight (mg):** | | **125.000** |

**Part B-2**

**General procedure for the preparation of inventive examples below employing a dry granulation process:**

1.0 Dispense the materials according to the indicated amounts.

2.0 Sift Bilastine, Microcrystalline Cellulose and Mannitol through suitable mesh.

3.0 Sift Sodium Starch Glycolate, Colloidal Anhydrous Silica, Magnesium Aluminometasilicate and Magnesium Stearate separately through suitable mesh.

4.0 Load the materials from the above steps 2.0 and 3.0 in blender and mix for sufficient time.

5.0 The blend from step 4.0 is roll compacted to prepare the granules.

6.0 The granules are milled to obtain suitable sized granules.

7.0 Load the granules from Step 6.0 into the blender. Add second portion of sifted Magnesium Stearate and Colloidal Anhydrous Silica into the blender and continue the blending for sufficient time.

8.0 Compress the tablets using lubricated blend from step 7.0.

The above compositions BIL/F1-7 may also be prepared using the components and/or amounts indicated above using the dry granulation process. The following composition BIL/F9 was obtained using the dry granulation process:

**Example BIL/F9:**

| S.No. | Ingredient | mg/tab | |
|---|---|---|---|
| | **Stage-A (Blending & Granulating)** | **Function** | **mg/tab** |
| 1 | Bilastine *(In house)* | API | 20.09 |
| 2 | Mannitol *Ph. Eur.* (Pearlitol® 200 SD) | Water-soluble filler | 73.00 |
| 3 | Cellulose, Microcrystalline *Ph. Eur.* (Avicel® PH 200) | Water-insoluble filler | 24.91 |
| 4 | Sodium Starch Glycolate *Ph. Eur.* (Explotab®) | Disintegrant | 1.50 |
| 5 | Silica, colloidal anhydrous *Ph. Eur.* (Aerosil® 200) | Glidant | 0.05 |
| 6 | Magnesium Aluminometasilicate *Ph. Eur.* (Neusilin® US2) | Glidant | 2.50 |
| 7 | Magnesium Stearate *Ph. Eur.* (Valtris) | Lubricant | 1.00 |
| | **Stage-B (Lubrication)** | | |
| 1 | Silica, colloidal anhydrous *Ph. Eur.* (Aerosil® 200 Pharma) | Glidant | 0.50 |
| 2 | Magnesium Stearate *Ph. Eur.* (Valtris) | Lubricant | 1.00 |
| | **Tablet Weight (mg):** | | **125.00** |

**Part C-1**

**Disintegration Study:**

The disintegration time of tablets was determined according to the US Pharmacopeia (USP) test for uncoated tablets in 37°C deionized water. The reported result is an average of 6 measurements. As can be determined from the data below, the disintegration time is short, and comparable between both the inventive and comparative examples.

| Batch | Disintegration time (sec) |
|---|---|
| **Bitosen® 20 mg (62033)** | 14-20 |
| **BIL/C1** | 8-11 |
| **BIL/C2** | 15-20 |
| **BIL/F1** | 15-25 |
| **BIL/F2** | 20-27 |
| **BIL/F3** | 25-35 |
| **BIL/F4** | 8-10 |
| **BIL/F5** | 36-64 |
| **BIL/F6** | 32-70 |

(continued)

| Batch | Disintegration time (sec) |
|---|---|
| **BIL/F7** | 32-79 |
| **BIL/F8** | 20-36 |

**Part C-2**

**Disintegration Study:**

The hardness of the dry granulated and compressed tablets (BIL/F9) was adjusted depending on the settings of the device used for compression. The disintegration time of tablets was then determined according to the US Pharmacopeia (USP) test for uncoated tablets in 37°C deionized water. Comparable results are obtained compared to the inventive examples above using the direct compression method.

**Table:**

| S. No. | Parameter | BIL/F9 (Hardness-50N) | BIL/F9 (Hardness-70N) | BIL/F9 (Hardness-90N) | BITOSEN® 20 mg Tabletten |
|---|---|---|---|---|---|
| 1 | Average weight (mg) | 126.4 | 125.2 | 125.3 | 124.5 |
| 2 | Thickness (mm) | 3.56-3.64 | 3.47-3.50 | 3.38-3.43 | 3.21-3.24 |
| 3 | Hardness (N) | 48-57 | 73-84 | 84-106 | 135-142 |
| 4 | Disintegration time (sec) | 19-27 | 30-42 | 50-74 | 14-20 |

**Part D**

**Dissolution Study:**

For the tablet formulations of inventive examples and comparative examples, the dissolution test was carried out according to the following dissolution test methods and conditions.

Dissolution conditions and methods:

The test was carried out using tablets at a paddle speed of 50 revolutions per minute (RPM) according to method 2 (Paddle) of dissolution test of the USP, using 900 mL of acetate buffer at pH 4.5. The temperature of the medium is maintained at 37°C ± 0.5°C using a water bath. Sample solutions were obtained at 5, 10, 15, 20, 30, 45 and 60 minutes after starting the test, and filtered through a 0.45 μm PVDF Millipore syringe filter.

Multimedia dissolution profiles are generated in the hydrochloric acid medium at pH 1.2 and in phosphate buffer solution at pH 6.8 also according to method 2 (Paddle) of dissolution test of the USP. Analogously, the dissolution is tested under the alternative conditions: 250 mL of acetate buffer at pH 4.5, method 2 (Paddle) with PEAK vessels at a paddle speed of 25RPM.

Dissolution analytical test method details:

**Equipment:**

- A High Performance Liquid Chromatography system with isocratic elution capability, a Spectrophotometric UV detector and an auto sampler (Waters Alliance 2695 separations module, Waters 2487 dual λ absorbance detector or equivalent).

- Data handling system (Waters Empower work station or equivalent).

- Analytical column: A stainless steel column 150 mm long, 4.6 mm internal diameter filled with octadecylsilyl silica particles as a stationary phase with size 3.5μm. (Use: Xterra RP18, 150 mm length, 4.6 mm internal diameter, 3.5μm particle size or equivalent).

- Dissolution Tester (Make: Electrolab, model TDT-08L or equivalent).

Preparation of analytical solutions:

**Buffer:** Prepare 10mm Di Potassium Hydrogen Phosphate Anhydrous. For example, transfer 1.76 gm of Di Potassium Hydrogen Phosphate Anhydrous in to beaker containing 1000 mL of Milli-Q- grade water. Adjusted pH to 6.8 with Diluted Orthophosphoric acid. Filter through 0.45μ micron or finer porosity membrane filter and degas.

**Mobile phase:** Buffer: Acetonitrile (65:35 v/v).

**Preparation of diluent:** Mix water and Acetonitrile in the ratio of 50:50 (v/v) and degas.

**Preparation of dissolution media:** Prepare acetate buffer solution pH 4.5 in purified water as mentioned in the Ph. Eur. 5.17.1 For example: Dissolve 29.9 g of Sodium acetate trihydrate and 16.6 mL of Acetic acid into a 10,000 mL beaker containing 8,000 mL of purified water. Dissolve and dilute to 10000 mL with water and mix. Adjust the pH to 4.5 if necessary, with Acetic acid or diluted Sodium hydroxide solution.

**Standard solution:** Prepare a solution containing 0.022 mg/mL of Bilastine in diluent. For example, weigh and transfer about 22 mg of Bilastine working standard into 50 mL clean, dry volumetric flask add about 10 mL of diluent and sonicate to dissolve. Further add 30mL of dissolution media and sonicate for 2 minutes. Make up the volume with Dissolution media. Dilute 5 mL to 100 mL with dissolution media. Prepare it in duplicate.

**Sample solution:** Set the parameters of instrument as mentioned above. Place one tablet each in six vessels containing the dissolution medium, which has been equilibrated at 37°C±0.5°C and start the dissolution tester. At the specified time interval withdraw sample solution from each vessel. Filter through 0.45μm syringe filter, discarding first few mL of filtrate.

HPLC chromatographic conditions:

- Column: Xterra RP18, 150mm length, 4.6mm internal diameter, 3.5μm particle size or equivalent

- Flow rate: 1.0 mL/min

- Detection: UV, 215 nm

- Injection Volume: 10 μL

- Data acquisition time: 5 minutes

- Pump mode: Isocratic

- Column temperature: 30° C

**Precautions during dissolution test**

Saturate the filter with about 10 mL of sample solution before collection of samples. Use prefilter at the end of dissolution cannula during sample collection in dissolution vessel. 0.45μm PVDF (Make: Millipore or Whatman). During the sample filtration avoid entrapment of air bubbles in to the filter. In case of dissolution profiles, use separate filter at each time point.

**Evaluation of system suitability**

Equilibrate the column and system at the initial composition for 30 minutes. Inject the dissolution media as blank into the liquid chromatographic system and record the chromatogram. Inject the STD-I solution, five times into the liquid chromatographic system and record the chromatogram. Symmetry factor should be not more than 2.0 for the Bilastine peak from the standard chromatogram. %RSD for Bilastine peak areas of five injections from STD-I should be not more than 2.0. Inject STD-II solution in duplicate into the liquid chromatographic system and record the chromatogram.

Calculate the similarity factor between two standard preparations. The similarity factor between two standard preparations should be in between 0.98 to 1.02.

**Calculation of Similarity factor**

$$\text{Similarity factor} = \frac{\text{Average area of STD-I}}{\text{Average area of STD-II}} \times \frac{\text{Weight of STD-II}}{\text{Weight of STD-I}}$$

**Procedure**

Inject the sample solution into the liquid chromatography and record the chromatogram.

Retention time of Bilastine is about 2.8 minutes.

**Calculation**

$$\%\ \text{Labelled amount as Bilastine} = \frac{At}{As} \times \frac{Ws}{50} \times \frac{5}{100} \times \frac{900}{Lc} \times P$$

where,

At : Area of peak corresponding to Bilastine in test solution chromatogram

As : Average area of peak corresponding to Bilastine obtained from STD-I chromatograms.

Ws : Weight of Bilastine working standard used for the preparation of STD-I (mg).

Lc : Label claim of Bilastine (mg).

P : % Potency of Bilastine working standard on as is basis.

**Table: Comparative dissolution profiles of Bilastine tablets 20 mg with Bitosen® 20 mg in Acetate buffer pH 4.5, Paddle-50 RPM, 900mL**

| | **Mean cumulative % labelled amount dissolved** | | | | |
|---|---|---|---|---|---|
| **Time in minutes** | **Bitosen® 20 mg Tabletten (Batch No. 62033)** | **BIL/C1** | **BIL/F5** | **BIL/F6** | **BIL/F7** |
| 5 | 56 | 57 | 68 | 53 | 55 |
| 10 | 77 | 65 | 82 | 74 | 70 |
| 15 | 86 | 68 | 88 | 85 | 79 |
| 20 | 90 | 70 | 93 | 90 | 84 |
| 30 | 94 | 72 | 95 | 94 | 90 |
| 45 | 96 | 74 | 97 | 96 | 93 |
| 60 | 97 | 75 | 97 | 97 | 93 |
| ***F2*** | - | *NC* | - | - | ***63*** |
| *NC = not calculated* | | | | | |

Dissolution of the inventive formulations of the present invention in Acetate buffer at pH 4.5 is comparable to the commercially available reference product (Bitosen ® 20 mg Tabletten).

The dissolution for the comparative example BIL/C1 is slower, and the complete drug release is not achieved even at 60 min time point (75% BIL/C1 vs. 97% for Bitosen® batch 62033).

**Table: Comparative dissolution profiles of Bilastine tablets 20 mg with Bitosen® 20 mg in Acetate buffer pH 4.5, Paddle with peak vessel - 25 RPM, 250mL**

| Time in minutes | Mean cumulative % labelled amount dissolved | | | | |
|---|---|---|---|---|---|
| | Bitosen® 20 mg Tabletten (Batch No. 53047) | BIL/C1 | BIL/C2 | BIL/F1 | BIL/F2 |
| 5 | 19 | 19 | 20 | 16 | 30 |
| 10 | 32 | 28 | 25 | 35 | 42 |
| 15 | 43 | 33 | 30 | 50 | 56 |
| 20 | 53 | 39 | 33 | 61 | 64 |
| 30 | 66 | 45 | 40 | 74 | 72 |
| 45 | 78 | 53 | 47 | 83 | 79 |
| 60 | 86 | 58 | 52 | 88 | 86 |

**Table: Comparative dissolution profiles of Bilastine tablets 20 mg with Bitosen® 20 mg in Phosphate buffer solution pH 6.8, Paddle-50 RPM, 900mL**

| Time in minutes | Mean cumulative % labelled amount dissolved | | |
|---|---|---|---|
| | Bitosen® 20 mg Tabletten (Batch No. 62033) | BIL/F5 | BIL/F7 |
| 5 | 42 | 54 | 45 |
| 10 | 60 | 71 | 59 |
| 15 | 72 | 77 | 65 |
| 20 | 79 | 80 | 70 |
| 30 | 86 | 86 | 76 |
| 45 | 90 | 90 | 81 |
| 60 | 92 | 92 | 84 |
| ***F2*** | - | ***56*** | ***58*** |

**Table: Comparative dissolution profiles of Bilastine tablets 20 mg with Bitosen® 20 mg in Hydrochloric acid media pH 1.2, Paddle-50 RPM, 900mL**

| Time in minutes | Mean cumulative % labelled amount dissolved | | |
|---|---|---|---|
| | Bitosen® 20 mg Tabletten (Batch No. 62033) | BIL/F5 | BIL/F7 |
| 5 | 82 | 93 | 80 |
| 10 | 96 | 98 | 88 |
| 15 | 100 | 99 | 90 |
| 20 | 101 | 99 | 91 |
| 30 | 101 | 99 | 93 |
| 45 | 101 | 99 | 94 |

(continued)

| Time in minutes | Mean cumulative % labelled amount dissolved | | |
|---|---|---|---|
| | Bitosen® 20 mg Tabletten (Batch No. 62033) | BIL/F5 | BIL/F7 |
| 60 | 102 | 99 | 94 |

As can be seen from the tables above, the inventive compositions are improved with respect to their dissolution times compared to the comparative examples when assessed using acetate buffer at pH 4.5. The presence of a water-soluble filler therefore enables the improvement of dissolution of polymorph 2, such that it is comparable to the composition comprising polymorph 1. The inventive examples show comparable dissolution profiles to the commercially available reference product (Bitosen® 20 mg Tabletten) when assessed in phosphate buffer of hydrochloric acid media at pH 6.8 and 1.2, respectively.

**Table: Comparative dissolution profiles of the formulation with Mannitol (Initial & 1 month- 40°C/75% RH in Alu-Alu blister) with Innovator in QC release media (Acetate buffer pH 4.5, Paddle, 50 RPM, 900mL)**

| Product/Batch No. | Mean Cumulative % Labelled Amount Dissolved | | | | | | | |
|---|---|---|---|---|---|---|---|---|
| | 5 min | 10 min | 15 min | 20 min | 30 min | 45 min | 60 min | *F2* |
| BITOSEN® 20mg Tabletten (B.No. 62033) | 56 | 77 | 86 | 90 | 94 | 96 | 97 | - |
| BIL/F5 (Initial) | 67 | 84 | 90 | 93 | 96 | 99 | 99 | *Not applicable* |
| BIL/F5 (1 Month-40°C/ 75%RH in Alu-Alu blister) | 48 | 71 | 79 | 82 | 91 | 94 | 97 | *59* |

As can be observed from the table above, the dissolution profile of the test formulation with Mannitol shows a slight (but otherwise acceptable) drop in the dissolution profile at the 10-20 minute time frame of the experiment, seen in the 1 month storage in Alu-Alu blister at 40°C/75% RH condition.

**Table: Comparative dissolution profiles of formulations with Talc (Initial & 1 Month-40°C/75% RH in Alu-Alu blister) with Innovator in QC release media (Acetate buffer pH 4.5, Paddle, 50 RPM, 900mL)**

| Product /Batch No. | Mean Cumulative % Labelled Amount Dissolved | | | | | | | |
|---|---|---|---|---|---|---|---|---|
| | 5 min | 10 min | 15 min | 20 min | 30 min | 45 min | 60 min | *F2* |
| BITOSEN® 20mg Tabletten (B.No. 62033) | 56 | 77 | 86 | 90 | 94 | 96 | 97 | - |
| BIL/F6 (Initial) | 53 | 74 | 85 | 90 | 94 | 96 | 97 | *Not applicable* |
| BIL/F6 (1 Month-40°C/ 75%RH in Alu-Alu blister) | 51 | 73 | 83 | 87 | 93 | 95 | 95 | *70* |
| BIL/F6 (2 Months-40°C/ 75%RH in Alu-Alu blister) | 50 | 73 | 80 | 84 | 87 | 89 | 90 | *61* |
| BIL/F6 (14 days, 60°C/ 40%RH, open exposure) | 47 | 76 | 87 | 90 | 93 | 96 | 97 | - |
| BIL/F7 (initial) | 55 | 70 | 79 | 84 | 90 | 93 | 93 | 63 |
| BIL/F7 (1 Month-40°C/ 75%RH in Alu-Alu blister) | 46 | 70 | 83 | 89 | 94 | 97 | 98 | 60 |
| BIL/F7 (14 days, 60°C/ 40%RH, open exposure) | 64 | 81 | 85 | 88 | 91 | 92 | 93 | *NC* |

As can be observed from the table above, the dissolution profile of the test formulations BIL/F6 and BIL/F7 with addition of Talc shows comparable dissolution profile after the 1 month - 40°C/75% RH (in Alu-Alu blister) conditioning of the tablet when compared to the fresh tablet.

An additional dissolution test was conducted for BIL/F8. Tablets according to the BIL/F8 composition described above were stored in the following conditions: 60°C / 40% RH for 14 days (open exposure). The dissolution profiles were found to be similar for initial tablets and for tablets stored at 60°C / 40% RH for 14 days (open exposure), as shown below:

**Table: Comparative dissolution profiles of bilastine tablets (BIL/F8) in QC release media (Acetate buffer pH 4.5, Paddle, 50 RPM, 900mL):**

| Time points | BIL/F8, Initial | BIL/F8, 60°C/40% RH (14 Days, open exposure) |
|---|---|---|
| 5 min | 82 | 88 |
| 10 min | 90 | 97 |
| 15 min | 93 | 99 |
| 20 min | 94 | 100 |
| 30 min | 95 | 100 |
| 45 min | 96 | 100 |
| 60 min | 96 | 100 |

Additional dissolution studies were conducted using the formulation prepared by dry granulation (BIL/F9), as shown in the table below. Comparable dissolution to the commercial preparation was obtained in acetate buffer. Similar results were obtained when using hydrochloric acid media pH 1.2 or phosphate buffer pH 6.8.

**Table: Comparative dissolution profiles of formulation BIL/F9 (Hardness-90N) with Innovator in QC release media (Acetate buffer pH 4.5, Paddle, 50 RPM, 900mL)**

| Product /Batch No. | Mean Cumulative % Labelled Amount Dissolved | | | | | | |
|---|---|---|---|---|---|---|---|
| | 5 min | 10 min | 15 min | 20 min | 30 min | 45 min | 60 min |
| BITOSEN® 20mg Tabletten (B.No. 62033) | 56 | 77 | 86 | 90 | 94 | 96 | 97 |
| BIL/F9 (Initial) | 51 | 76 | 88 | 93 | 97 | 99 | 100 |
| BIL/F9 (1 Month-40°C/75%RH in Alu-Alu blister) | 63 | 83 | 91 | 95 | 97 | 98 | 98 |
| BIL/F9 (1 Month-25°C/60%RH in Alu-Alu blister) | 54 | 78 | 88 | 93 | 97 | 98 | 98 |
| BIL/F9 (14 days- 60°C/40%RH open) | 62 | 81 | 90 | 94 | 97 | 99 | 99 |

**Table: Comparative dissolution profiles of Bilastine tablets 20 mg with Bitosen® 20 mg in Phosphate buffer solution pH 6.8, Paddle-50 RPM, 900mL**

| Time in minutes | Mean cumulative % labelled amount dissolved | | |
|---|---|---|---|
| | Bitosen® 20 mg Tabletten (Batch No. 62033) | BIL/F9 (batch 1) | BIL/F9 (batch 2) |
| 5 | 42 | 35 | 34 |
| 10 | 60 | 54 | 52 |
| 15 | 72 | 65 | 63 |
| 20 | 79 | 72 | 71 |

(continued)

| Time in minutes | Mean cumulative % labelled amount dissolved | | |
|---|---|---|---|
| | Bitosen® 20 mg Tabletten (Batch No. 62033) | BIL/F9 (batch 1) | BIL/F9 (batch 2) |
| 30 | 86 | 82 | 80 |
| 45 | 90 | 89 | 88 |
| 60 | 92 | 93 | 91 |
| ***F2*** | **-** | ***62*** | ***94*** |

**Table: Comparative dissolution profiles of Bilastine tablets 20 mg with Bitosen® 20 mg in Hydrochloric acid media pH 1.2, Paddle-50 RPM, 900mL**

| Time in minutes | Mean cumulative % labelled amount dissolved | | |
|---|---|---|---|
| | Bitosen® 20 mg Tabletten (Batch No. 62033) | BIL/F9 (batch 1) | BIL/F9 (batch 2) |
| 5 | 82 | 83 | 89 |
| 10 | 96 | 94 | 94 |
| 15 | 100 | 96 | 95 |
| 20 | 101 | 97 | 95 |
| 30 | 101 | 99 | 96 |
| 45 | 101 | 100 | 97 |
| 60 | 102 | 101 | 97 |

**Table: Comparative dissolution profiles of Bilastine tablets 20 mg with Bitosen® 20 mg in acetate buffer pH 4.5, Paddle-50 RPM, 900mL**

| Time in minutes | Mean cumulative % labelled amount dissolved | | |
|---|---|---|---|
| | Bitosen® 20 mg Tabletten (Batch No. 62033) | BIL/F9 (batch 1) | BIL/F9 (batch 2) |
| 5 | 56 | 48 | 51 |
| 10 | 77 | 75 | 76 |
| 15 | 86 | 86 | 88 |
| 20 | 90 | 92 | 93 |
| 30 | 94 | 96 | 97 |
| 45 | 96 | 98 | 99 |
| 60 | 97 | 98 | 100 |

**Comparative dissolution profiles of Bilastine tablets BIL/F9 (Batch 1)**

Bilastine 20 mg Tablets were loaded in to the below mentioned conditions and tablets were analyzed for Dissolution test at respective time intervals.

**Table:**

| Time in minutes | BIL/A-115 Initial | 70°C/75% RH_Day 2 | 80°C/40% RH_Day 2 | 50 °C/75% RH_Day 14 | 60 °C/40% RH_Day 14 | 60 °C/75% RH_Day 14 | 70 °C/5% RH_Day 14 |
|---|---|---|---|---|---|---|---|
| 5 | 48 | 50 | 59 | 36 | 66 | 37 | 67 |
| 10 | 75 | 70 | 75 | 61 | 81 | 63 | 83 |
| 15 | 86 | 83 | 85 | 76 | 87 | 78 | 89 |
| 20 | 92 | 91 | 92 | 84 | 90 | 86 | 92 |
| 30 | 96 | 96 | 97 | 91 | 93 | 93 | 94 |
| 45 | 98 | 99 | 98 | 93 | 94 | 95 | 95 |
| 60 | 98 | 100 | 99 | 94 | 94 | 95 | 95 |

As is evident from the table above, complete dissolution release was observed when the open tablets were exposed across ASAP conditions. The influence of humidity was not significant on the formulation (BIL/F9) with respect to release, in particular when the dissolution results are compared with compositions (BIL/F6, BIL/F7 and BIL/F8).

**Results of related substances by HPLC (BIL/F9):**

Bilastine 20 mg Tablets were loaded in to the below mentioned conditions and tablets were analyzed for related substances by HPLC test at respective time interval.

**Table:**

| Name of the impurity | Initial | 70 °C/75% RH | 80 °C/40% RH | 50 °C/75% RH | 60 °C/40% RH | 60 °C/75% RH | 70 °C/5% RH |
|---|---|---|---|---|---|---|---|
| | | 2 Days | 2 Days | 14 Days | 14 Days | 14 Days | 14 Days |
| N-Oxide Impurity | 0.02 | 0.02 | 0.02 | 0.02 | 0.03 | 0.03 | 0.03 |
| Single maximum (1.99 | 0.08 | 0.07 | 0.07 | 0.07 | 0.07 | 0.07 | 0.07 |
| Total Impurity | 0.20 | 0.25 | 0.24 | 0.24 | 0.25 | 0.25 | 0.26 |

As is evident from the table above, there is no significant influence of temperature and humidity observed on related substances of Bilastine in selected composition of BIL/F9.

**Part E**

**Blend Uniformity and Content Uniformity of Tablets:**

The robustness and reproducibility of the direct compression process was evaluated by testing the blend uniformity and content uniformity of tablets produced according to the invention using different blending parameters. The formulation according to unit formula below was employed. Nine batches were prepared using varying combinations of usual blending parameters: 10-20 min for pre-lubrication time, 3-7 min for the lubrication time, and 10-30 rpm for the blender speed. No difficulties like sticking or poor blend flow were observed in any batches below.

**Table: Batches prepared for assessment of blend and content uniformity Unit formula of batches BIL/F10-F19:**

| S.No. | Ingredient | mg/tab | |
|---|---|---|---|
| | **Stage-A (Sifting & Blending)** | **Function** | **mg/tab** |
| 1 | Bilastine *(In house)* | API | 20.000 |
| 2 | Mannitol *Ph. Eur.* (Pearlitol® 200 SD) | Water-soluble filler | 73.000 |

(continued)

| S.No. | Ingredient | mg/tab | |
|---|---|---|---|
| | **Stage-A (Sifting & Blending)** | **Function** | **mg/tab** |
| 3 | Cellulose, Microcrystalline *Ph. Eur.* (Avicel® PH 200) | Water-insoluble filler | 28.750 |
| 4 | Crospovidone *Ph. Eur.* (Polyplasdone® XL-10) | Disintegrant | 0.500 |
| 5 | Silica, colloidal anhydrous *Ph. Eur.* (Aerosil® 200) | Glidant | 1.000 |
| | **Stage-B (Lubrication)** | | |
| 1 | Magnesium Stearate *Ph. Eur.* (Ferro) | Lubricant | 1.750 |
| | **Tablet Weight (mg):** | | **125.000** |

***Blending Parameters:***

| Batch no. | Pre-lubrication time (min) | Lubrication time (min) | Blender speed (rpm) |
|---|---|---|---|
| **BIL/F10** | 10 | 3 | 10 |
| **BIL/F11** | 20 | 7 | 10 |
| **BIL/F12** | 15 | 7 | 20 |
| **BIL/F13** | 15 | 5 | 20 |
| **BIL/F14** | 20 | 5 | 20 |
| **BIL/F15** | 10 | 5 | 20 |
| **BIL/F16** | 15 | 3 | 20 |
| **BIL/F17** | 20 | 3 | 30 |
| **BIL/F18** | 10 | 7 | 30 |
| **BIL/F19** | 15 | 5 | 30 |

To ensure the consistency of dosage units, each unit in a batch should have an active substance content within a narrow range around the label claim. Dosage units are defined as dosage forms containing a single dose or a part of a dose of an active substance in each dosage unit. The term "Uniformity of dosage unit" is defined as the degree of uniformity in the amount of the active substance among dosage units. Therefore, the requirements of this regulation can be applied to any active substance being comprised in dosage units containing one or more active substances. The uniformity of dosage forms (by content uniformity) was assessed according to European Pharmacopeia 2.9.40. The assay (%) of 10 individual samples (tablets or locations in blend) was detected by HPLC analysis according to the following HPLC conditions:

**Instrumentation**

- A High Performance Liquid Chromatography system with isocratic elution capability, a Spectrophotometric UV detector and an auto sampler (Waters Alliance 2695 separations module, Waters 2489 dual $\lambda$ absorbance detector or equivalent).
- Data handling system (Waters Empower work station or equivalent).
- Analytical column: A stainless steel column 150 mm long, 4.6 mm internal diameter filled with octadecylsilyl silica particles as a stationary phase with size 3.5 $\mu$m. (Use: XBridge Shield RP18, 150 mm length, 4.6 mm internal

diameter, 3.5μm particle size or equivalent.)

**Reagents**

- Water: HPLC / Milli-Q- grade
- Acetonitrile: HPLC grade (Make: Merck)
- Di Potassium hydrogen Phosphate: Emparta grade (Make: Merck)
- Ortho phosphoric acid: AR grade (Make: Merck)

*Note: All the reagents/solvents can be used above mentioned make or equivalent*

**Preparation of Mobile phase**

- **Buffer:** Prepare 10mm Di Potassium Hydrogen Phosphate Anhydrous. For example, transfer 1.76 gm of Di Potassium Hydrogen Phosphate Anhydrous in to beaker containing 1000 mL of Milli-Q- grade water. Adjusted pH to 6.8 with Diluted Orthophosphoric acid Filter through 0.45μ micron or finer porosity membrane filter and degas.
- **Mobile phase:** Buffer: Acetonitrile (70:30 V/v).
- **Preparation of diluent:** Mix water and Acetonitrile in the ratio of 50:50 (v/v) and degas.

**Chromatographic conditions**

- Column : XBridge Shield RP18, 150mm length, 4.6mm internal diameter, 3.5μm particle size or equivalent.
- Flow rate: 1.0 mL/min
- Detection UV, 215 nm
- Injection Volume: 10 μL
- Data acquisition time: 8 minutes
- Column temperature: 30°C
- Pump mode: Isocratic

**Preparation of solutions**

**Standard solution:** Prepare a solution containing 0.08 mg/mL of Bilastine in diluent.

For example, weigh accurately 50 mg of Bilastine working standard into a 50 mL clean, dry volumetric flask. Add 15 mL of diluent and sonicate to dissolve. Make up to volume with diluent and mix. Dilute 4.0 mL of this solution to 50 mL with diluent. Label this working standard solution as the STD-I. Prepare the working standard solution in duplicate and label it as STD-II.

**Sample solution:** For Example: Carefully transfer entire amount of sample from glass vial into a 200 mL volumetric flask by using funnel. Rinse the vial completely with water and transfer the rinsing volume into the same volumetric flask. Repeat the rinsing of vial procedure at least 3 times so that entire sample will transfer into volumetric flask. Add 50 mL diluent shake for 20 minutes at 150 RPM. Add 50 mL diluent and sonicate for 15 minutes with intermittent shaking, make up the volume with diluent. Shake well and filter through 0.45 micron membrane filter. Further dilute 5

mL to 20 mL with diluent.

**Evaluation of system suitability**

- Equilibrate the column and system at the initial composition for 30 minutes.
- Inject the diluent as blank into the liquid chromatographic system and record the chromatogram.
- Inject the STD-I solution, five times into the liquid chromatographic system and record the chromatogram.
- Symmetry factor should be not more than 2.0 for the Bilastine peak from the standard chromatogram. Retention time of Bilastine peak is about 4.7 minutes.
- %RSD for Bilastine peak areas of five injections from STD-I should be not more than 2.0.
- Inject STD-II solution in duplicate into the liquid chromatographic system and record the chromatogram.
- Calculate the similarity factor between two standard preparations. The similarity factor between two standard preparations should be in between 0.98 to 1.02.

**Calculation of Similarity factor**

$$\text{Similarity factor} = \frac{\text{Average area of STD-I}}{\text{Average area of STD-II}} \times \frac{\text{Weight of STD-II}}{\text{Weight of STD-I}}$$

**Calculation**

$$\text{Assay (\% Label claim)} = \frac{At}{As} \times \frac{Ws}{50} \times \frac{4}{50} \times \frac{200}{Wt} \times \frac{20}{5} \times \frac{LQ}{LC} \times P$$

Where,

At : Area of peak corresponding to Bilastine in test solution chromatogram
As : Average area of peak corresponding to Bilastine obtained from STD-I
Ws : Weight of Bilastine standard used for the preparation of STD-I (mg).
Wt : Weight of sample (mg)
LC : Label claim of Bilastine (mg)
P : % Assay of Bilastine working standard on as is basis.
LQ : Label Quantity of Bilastine (mg)

The results are presented in tables below.

**Table: Results from assessment of blend uniformity and blend assay**

| Uniform ity of blend in batches | BIL/F10 | BIL/F11 | BIL/F12 | BIL/F13 | BIL/F14 | BIL/F15 | BIL/F16 | BIL/F17 | BIL/F18 | BIL/F19 |
|---|---|---|---|---|---|---|---|---|---|---|
| Location-1 | 98.8 | 98.9 | 102.6 | 97.1 | 98.2 | 102.6 | 100.6 | 102.0 | 103.3 | 100.7 |
| Location-2 | 99.8 | 97.5 | 97.0 | 98.5 | 99.1 | 103.8 | 101.2 | 100.3 | 99.4 | 99.8 |
| Location-3 | 100.1 | 97.2 | 102.9 | 99.8 | 102.7 | 102.2 | 100.3 | 99.8 | 99.6 | 100.0 |
| Location-4 | 98.7 | 97.0 | 97.0 | 100.1 | 100.5 | 103.2 | 99.5 | 101.6 | 99.0 | 100.6 |
| Location-5 | 99.2 | 98.3 | 95.9 | 101.3 | 100.5 | 103.5 | 98.1 | 99.9 | 99.1 | 101.1 |
| Location-6 | 98.9 | 98.0 | 98.4 | 99.9 | 98.7 | 103.8 | 100.9 | 100.3 | 100.3 | 102.7 |
| Location-7 | 97.6 | 97.4 | 97.6 | 101.3 | 99.3 | 101.6 | 100.0 | 100.2 | 102.9 | 98.0 |
| Location-8 | 98.6 | 98.1 | 97.6 | 99.0 | 98.2 | 102.7 | 99.7 | 101.4 | 99.4 | 98.1 |
| Location-9 | 98.0 | 99.4 | 100.3 | 100.2 | 103.7 | 101.3 | 100.2 | 98.7 | 99.6 | 100.4 |
| Location-10 | 99.1 | 97.1 | 101.0 | 99.9 | 96.5 | 99.4 | 99.7 | 99.1 | 101.4 | 100.2 |
| **Mean** | **98.9** | **97.9** | **99.0** | **99.7** | **99.7** | **102.4** | **100.0** | **100.3** | **100.4** | **100.2** |
| **%RSD** | **0.76** | **0.82** | **2.51** | **1.26** | **2.18** | **1.33** | **0.87** | **1.06** | **1.58** | **1.37** |
| **Assay** | **99.30** | **97.40** | **97.70** | **97.70** | **96.90** | **97.3** | **99.3** | **97.4** | **97.9** | **99.4** |

**Table: Results from assessment of content uniformity and assay of tablets**

| Uniform ity of dosage units | BIL/F10 | BIL/F11 | BIL/F12 | BIL/F13 | BIL/F14 | BIL/F15 | BIL/F16 | BIL/F17 | BIL/F18 | BIL/F19 |
|---|---|---|---|---|---|---|---|---|---|---|
| Tablet-1 | 101.2 | 97.4 | 97.8 | 99.1 | 97.1 | 99.8 | 97.7 | 99.2 | 99.1 | 95.5 |
| Tablet-2 | 99.6 | 96.8 | 97.5 | 100.3 | 97.3 | 98.6 | 100 | 98.2 | 99.6 | 97.3 |
| Tablet-3 | 100.4 | 96.4 | 97.9 | 100.4 | 93.6 | 99.9 | 98.0 | 97.6 | 99.0 | 95.9 |
| Tablet-4 | 99.4 | 96.3 | 95.6 | 99.8 | 99.7 | 95.8 | 99.8 | 98.6 | 98.6 | 98.2 |
| Tablet-5 | 98.8 | 97.3 | 97.9 | 101.2 | 100.8 | 101.2 | 98.9 | 97.5 | 98.6 | 96.4 |
| Tablet-6 | 100.9 | 96.8 | 96.9 | 99.9 | 98.0 | 98.5 | 96.7 | 96.6 | 100.0 | 95.7 |
| Tablet-7 | 99.6 | 94.6 | 96.7 | 99.8 | 99.9 | 99.5 | 99.6 | 97.9 | 96.3 | 99.9 |
| Tablet-8 | 100.8 | 96.4 | 99.0 | 102.9 | 97.3 | 99.8 | 100.4 | 98.6 | 98.3 | 95.1 |
| Tablet-9 | 101.4 | 97.6 | 99.7 | 101.2 | 99.2 | 99.2 | 99.4 | 98.2 | 98.2 | 95 |
| Tablet-10 | 99.7 | 95.7 | 98.8 | 100.1 | 98.7 | 99.4 | 98.7 | 96.0 | 98.2 | 95.3 |
| **Mean** | **100.2** | **96.5** | **97.8** | **100.5** | **98.2** | **99.2** | **98.9** | **97.8** | **98.6** | **96.4** |
| **%RSD** | **0.87** | **0.92** | **1.23** | **1.06** | **2.07** | **1.42** | **1.17** | **0.99** | **1.02** | **1.65** |
| **Assay** | **100.1** | **99.1** | **101.3** | **100.2** | **99.1** | **100.3** | **99.6** | **99.2** | **99.3** | **97.6** |

As can be observed from the data presented above, the content uniformity is found to be within the specification limits according to European Pharmacopeia 2.9.40 independent of changes in the blending parameters (pre-lubrication time, lubrication time, and blender speed).

Furthermore, both the assay of tablets and the average amount of API contained per tablet (as described above under "Mean") demonstrate values of 97.6-101.8% and 96.4-100.5%, correspondingly, clearly satisfying the standard requirements of 95-105%.

## Part F

### Comparison to Example 10D of WO 2017/017301:

The table below shows the composition employed in additional comparative experimental work using a composition corresponding essentially to Example 10D of WO 2017/017301, which also employs the polymorphic form 2 of Bilastine.

**Table:**

| S.No. | Ingredient | mg/tab |
|---|---|---|
| | **Stage - A (Sifting & Blending)** | **mg/tab** |
| 1 | Bilastine Form-2 (*In house*) | 20.090 |
| 2 | Cellulose, Microcrystalline *Ph.Eur.* (Comprecel® M102 D+) | 98.030 |
| 3 | Sodium Starch Glycolate *Ph.Eur.* (Explotab®) | 5.000 |
| 4 | Silica, colloidal anhydrous *Ph.Eur.* (Aerosil® 200 Pharma) | 0.630 |
| | **Stage - B (Lubrication)** | |
| 1 | Magnesium Stearate *Ph. Eur.* (Valtris) | 1.250 |
| | **Tablet Weight (mg):** | **125.000** |

### Manufacturing procedure employed for producing a formulation corresponding essentially to Example 10D of WO 2017/017301 (direct compression process):

1.0 Dispense the materials according to the above indicated amounts.

2.0 Co-sift Bilastine and Cellulose, Microcrystalline through suitable size mesh.

3.0 Sift Sodium Starch Glycolate and Colloidal Anhydrous Silica separately through suitable mesh.

4.0 Load the materials from steps 2.0 and 3.0 in blender and mix for sufficient time.

5.0 Sift Magnesium Stearate through suitable size mesh and add it to the blend from step 4.0 and continue the blending for sufficient time.

6.0 Compress the tablets on a rotary tablet compression machine using the blend 5.0.

**Table: Comparative dissolution profiles of formulation comparison of Example 10D of WO 2017/017301 with commercially available compositions in release media (Acetate buffer pH 4.5, Paddle, 50 RPM, 900mL)**

| Product/Batch No. | Mean Cumulative % Labelled Amount Dissolved | | | | | | |
|---|---|---|---|---|---|---|---|
| | 5 min | 10 min | 15 min | 20 min | 30 min | 45 min | 60 min |
| BITOSEN® 20mg Tabletten (B.No. 62033) | 56 | 77 | 86 | 90 | 94 | 96 | 97 |

(continued)

| Product/Batch No. | Mean Cumulative % Labelled Amount Dissolved | | | | | | |
|---|---|---|---|---|---|---|---|
| | 5 min | 10 min | 15 min | 20 min | 30 min | 45 min | 60 min |
| Example 10D of WO 2017/017301 | 66 | 77 | 80 | 83 | 84 | 86 | 87 |

As can be seen from the table above, the BITOSEN® 20mg Tablets (B.No. 62033), as an example of the commercially available formulation, showed improved dissolution times compared to the comparative examples according to Example 10D of WO 2017/017301 when assessed using acetate buffer at pH 4.5. With reference to the additional dissolution studies disclosed above in Part D, the examples according to the present invention show comparable dissolution profiles to the commercially available reference product (Bitosen® 20 mg) when assessed in acetate buffer. As such, it can be drawn from these data that the compositions of the present invention, as defined by the additional presence of a water-soluble filler, show improved solubility over alternative formulations (such as Example 10D of WO 2017/017301) comprising polymorph form 2 of Bilastine.

## Part G

**Polymorphic Stability:**

Additional analysis has also demonstrated a high stability of the polymorphic form 2 in the compositions of the present invention. The assessment of polymorphic stability of the API in tablets produced as described above via powder X-ray diffraction analysis shows that only polymorphic form 2 is present.

Compositions according to BIL/F5, BIL/F7, BIL/F8 and BIL/F9 were assessed after storage (open exposure or in Alu-Alu blister) at various temperature and humidity conditions.

**Table:**

| S.No | Batch No | Condition | API polymorphic form identification by XRD |
|---|---|---|---|
| 1 | BIL/F5 | Initial | Form 2 |
| 2 | BIL/F5 | 40°C/75%RH, 1 month in Alu-Alu blister | Form 2 |
| 3 | BIL/F7 | Initial | Form 2 |
| 4 | BIL/F7 | 40°C/75%RH, 1 month in Alu-Alu blister | Form 2 |
| 5 | BIL/F8 | Initial | Form 2 |
| 6 | BIL/F8 | 70°C/75%RH 2 Days open exposure | Form 2 |
| 7 | BIL/F8 | 50°C/75%RH 14 Days open exposure | Form 2 |
| 8 | BIL/F9 | Initial | Form 2 |
| 9 | BIL/F9 | 40°C/75%RH, 1 month in Alu-Alu blister | Form 2 |
| 10 | BIL/F9 | 70°C/75%RH 2 Days open exposure | Form 2 |

Exemplary powder X-ray diffraction results are demonstrated in the figures below (Figures 1-11).

The powder X-ray diffraction analysis of Bilastine API Form 1 (Fig. 1) and Form 2 (Fig. 2) is done according to below "XRD method D1", whilst "XRD method V2" is used for the powder X-ray diffraction analysis of all tablet samples (Figures 3-11).

XRD method D1:

The qualitative PXRD measurements were performed using the X'Pert MPD system, LLF x-ray source with CuKa radiation, Soller collimators of 0.02, 0.04 rad, divergent slit ½ deg, powder load was 0.4 g for API samples. The radiation used - CuKa (8keV); 40kV-40mA; measurement range 0-40 degrees, measurement time per scan - 2.5 hours, step = 0.013 degrees. During the measurements the sample was rotated at speed 4s/revolution to improve counting statistic.

XRD method V2:

A summary of XRD method parameters is described below.

| | |
|---|---|
| Model/ Make: | Bruker AXS/D8 Advance |
| X-Ray Tube: | Copper K$\alpha$1, 1.5406 Å |
| Detector: | Lynx Eye |
| K$\beta$ Filter: | Nickel |
| 2$\theta$ Start: | 3.0° |
| 2$\theta$ End: | 40.0° |
| Step Size: | 0.016° |
| Current: | 40 mA |
| Voltage: | 40 kV |
| Scan Type: | Locked coupled and continuous |
| Spinning: | 30 rpm |
| Divergence Slit: | 0.3° |
| Receiving Slit: | 3 mm |
| Total Time: | 1 Hr |

*Sample preparation:*

Take four tablets, grind to fine powder using neat and clean mortar and pestle.

Fill the fine powder in the round cavity of the sample holder and smooth the surface with glass plate/slide to obtain smooth surface. The sample surface should be in parallel to holder surface. Clean the outer edges of the holder with tissue paper to avoid sample contaminations.

Place the prepared sample holder on the sample stage of the XRD instrument and analyze as per the above method at room temperature.

## Claims

1. A pharmaceutical composition in the form of a tablet, comprising a) a crystalline form of bilastine, wherein the crystalline form has characteristic peaks at 6.53, 15.24, 15.86, and 18.07 $\pm$ 0.2 degrees 2-theta in a powder X-ray diffraction pattern, b) a water-soluble filler, and optionally c) a water-insoluble filler, wherein the water-soluble filler is a sugar, and wherein the composition is prepared by dry granulation of a blend of the components comprising a), b) and optionally c), and compression of the granules to a tablet, or direct compression of a blend of the components comprising a), b) and optionally c).

2. The pharmaceutical composition according to claim 1, wherein said composition is prepared by dry granulation comprising blending of the components of claim 1, roller compaction and granulation of the blend, milling the granules, lubricating the granules and subsequent compression of the granules to a tablet.

3. The pharmaceutical composition according to claim 1 or 2, wherein the composition further comprises a disintegrant, a glidant and/or a lubricant.

4. The pharmaceutical composition according to any one of the preceding claims, wherein the water-soluble filler is mannitol.

5. The pharmaceutical composition according to any one of the preceding claims, wherein the water-soluble filler is lactose.

6. The pharmaceutical composition according to any one of the preceding claims, wherein the water-insoluble filler is microcrystalline cellulose.

7. The pharmaceutical composition according to any one of the preceding claims, wherein the composition comprises a disintegrant selected from sodium starch glycolate, low-substituted hydroxypropylcellulose, pregelatinized starch,

or crospovidone, preferably sodium starch glycolate or crospovidone.

8. The pharmaceutical composition according to any one of the preceding claims, wherein the composition comprises a glidant, which is silicon dioxide, preferably as colloidal anhydrous silica, magnesium aluminometasilicate and/or talc, preferably a combination of silicon dioxide and magnesium aluminometasilicate.

9. The pharmaceutical composition according to any one of the preceding claims, wherein the composition comprises a lubricant, which is magnesium stearate.

10. The pharmaceutical composition according to any one of the preceding claims, comprising a crystalline form of bilastine, wherein the crystalline form has characteristic peaks at 6.53, 15.24, 15.86, and 18.07 ± 0.2 degrees 2-theta in a powder X-ray diffraction pattern, a water-soluble filler, a water-insoluble filler, a disintegrant, a glidant, and a lubricant, wherein the water-soluble filler is D-mannitol and the glidant is colloidal anhydrous silica or a combination of silicon dioxide and magnesium aluminometasilicate.

11. The pharmaceutical composition according to claim 3, wherein the water-soluble filler is D-mannitol, the water-insoluble filler is microcrystalline cellulose, the disintegrant is sodium starch glycolate, the glidant is colloidal anhydrous silica or a combination of colloidal anhydrous silica and magnesium aluminometasilicate, and the lubricant is magnesium stearate.

12. The pharmaceutical composition according to any one of the preceding claims, wherein the tablet is a coated or uncoated immediate-release tablet, preferably uncoated.

13. The pharmaceutical composition according to any one of the preceding claims for use as a medicament in the treatment of allergic rhino-conjunctivitis and/or urticaria.

14. Method of preparing a pharmaceutical composition in the form of a tablet, comprising:

a. dry granulation of a blend and compression of the granules to a tablet, wherein the blend comprises a) a crystalline form of bilastine, wherein the crystalline form has characteristic peaks at 6.53, 15.24, 15.86, and 18.07 ± 0.2 degrees 2-theta in a powder X-ray diffraction pattern, b) a water-soluble filler and optionally c) a water-insoluble filler, wherein the water-soluble filler is a sugar, preferably wherein the method comprises blending of the components a), b) and optionally c), roller compaction and granulation of the blend, milling the granules, lubricating the granules and subsequent compression of the granules to a tablet; or
b. direct compression of a blend to a tablet, wherein the blend comprises a) a crystalline form of bilastine, wherein the crystalline form has characteristic peaks at 6.53, 15.24, 15.86, and 18.07 ± 0.2 degrees 2-theta in a powder X-ray diffraction pattern, b) a water-soluble filler and optionally c) a water-insoluble filler, wherein the water-soluble filler is a sugar.

## Patentansprüche

1. Pharmazeutische Zusammensetzung in Form einer Tablette, umfassend a) eine kristalline Form von Bilastin, wobei die kristalline Form charakteristische Peaks bei 6,53, 15,24, 15,86 und 18,07 ± 0,2 Grad 2-Theta in einem Pulver-Röntgenbeugungsmuster aufweist, b) einen wasserlöslichen Füllstoff und gegebenenfalls c) einen wasserunlöslichen Füllstoff, wobei der wasserlösliche Füllstoff ein Zucker ist, und wobei die Zusammensetzung durch eine Trockengranulierung einer Mischung der Komponenten umfassend a), b), und ggf. c) und eine Verpressung des Granulats zu einer Tablette oder durch die Direktverpressung einer Mischung der Komponenten umfassend a), b), und ggf. c), hergestellt wird.

2. Pharmazeutische Zusammensetzung nach Anspruch 1, wobei die Zusammensetzung durch eine Trockengranulierung hergestellt wird, umfassend das Mischen der Komponenten nach Anspruch 1, Walzenkompaktierung und Granulierung der Mischung, Mahlen des Granulats, Schmieren des Granulats und anschließendes Verpressen des Granulats zu einer Tablette.

3. Pharmazeutische Zusammensetzung nach Anspruch 1 oder 2, wobei die Zusammensetzung ferner ein Sprengmittel, ein Fließregulierungsmittel und / oder ein Schmiermittel umfasst.

4. Pharmazeutische Zusammensetzung nach einem der vorhergehenden Ansprüche, wobei der wasserlösliche Füllstoff Mannitol ist.

5. Pharmazeutische Zusammensetzung nach einem der vorhergehenden Ansprüche, wobei der wasserlösliche Füllstoff Lactose ist.

6. Pharmazeutische Zusammensetzung nach einem der vorhergehenden Ansprüche, wobei der wasserunlösliche Füllstoff mikrokristalline Cellulose ist.

7. Pharmazeutische Zusammensetzung nach einem der vorhergehenden Ansprüche, wobei die Zusammensetzung ein Sprengmittel umfasst, ausgewählt aus Natriumstärkeglykolat, niedrigsubstituierter Hydroxypropylcellulose, vorgelatinierter Stärke oder Crospovidon, vorzugsweise Natriumstärkeglykolat oder Crospovidon.

8. Pharmazeutische Zusammensetzung nach einem der vorhergehenden Ansprüche, wobei die Zusammensetzung ein Fließregulierungsmittel umfasst, das Siliciumdioxid ist, vorzugsweise als kolloidales wasserfreies Siliciumdioxid, Magnesiumaluminiummetasilicat und/oder Talk, vorzugsweise eine Kombination von Siliciumdioxid und Magnesiumaluminiummetasilicat.

9. Pharmazeutische Zusammensetzung nach einem der vorhergehenden Ansprüche, wobei die Zusammensetzung ein Schmiermittel umfasst, das Magnesiumstearat ist.

10. Pharmazeutische Zusammensetzung nach einem der vorhergehenden Ansprüche, umfassend eine kristalline Form von Bilastin, wobei die kristalline Form charakteristische Peaks bei 6,53, 15,24, 15,86 und 18,07 ± 0,2 Grad 2-Theta in einem Pulver-Röntgenbeugungsmuster aufweist, einen wasserlöslichen Füllstoff, einen wasserunlöslichen Füllstoff, ein Sprengmittel, ein Fließregulierungsmittel und ein Schmiermittel, wobei der wasserlösliche Füllstoff D-Mannitol und das Fließregulierungsmittel kolloidales wasserfreies Siliciumdioxid oder eine Kombination aus Siliciumdioxid und Magnesiumaluminiummetasilicat ist.

11. Pharmazeutische Zusammensetzung nach Anspruch 3, wobei der wasserlösliche Füllstoff D-Mannitol, der wasserunlösliche Füllstoff mikrokristalline Cellulose, das Sprengmittel Natriumstärkeglykolat, das Fließregulierungsmittel kolloidales wasserfreies Siliciumdioxid oder eine Kombination aus kolloidalem wasserfreiem Siliciumdioxid und Magnesiumaluminiummetasilicat, und das Schmiermittel Magnesiumstearat ist.

12. Pharmazeutische Zusammensetzung nach einem der vorhergehenden Ansprüche, wobei die Tablette eine beschichtete oder unbeschichtete Tablette mit sofortiger Freisetzung ist, vorzugsweise eine unbeschichtete.

13. Pharmazeutische Zusammensetzung nach einem der vorhergehenden Ansprüche zur Verwendung als Medikament bei der Behandlung von allergischer Rhino-Konjunktivitis und/oder Urtikaria.

14. Verfahren zur Herstellung einer pharmazeutischen Zusammensetzung in Form einer Tablette, umfassend:

a. Trockengranulierung einer Mischung und Verpressung des Granulats zu einer Tablette, wobei die Mischung a) eine kristalline Form von Bilastin, wobei die kristalline Form charakteristische Peaks bei 6,53, 15,24, 15,86 und 18,07 ± 0,2 Grad 2-Theta in einem Pulver-Röntgenbeugungsmuster aufweist, b) einen wasserlöslichen Füllstoff und gegebenenfalls c) einen wasserunlöslichen Füllstoff umfasst, wobei der wasserlösliche Füllstoff ein Zucker ist, wobei das Verfahren vorzugsweise das Mischen der Komponenten a), b), und ggf. c), Walzenkompaktierung und Granulierung der Mischung, Mahlen des Granulats, Schmieren des Granulats und anschließendes Verpressen des Granulats zu einer Tablette umfasst; oder
b. Direktverpressung einer Mischung zu einer Tablette, wobei die Mischung a) eine kristalline Form von Bilastin, wobei die kristalline Form charakteristische Peaks bei 6,53, 15,24, 15,86 und 18,07 ± 0,2 Grad 2-Theta in einem Pulver-Röntgenbeugungsmuster aufweist, b) einen wasserlöslichen Füllstoff und gegebenenfalls c) einen wasserunlöslichen Füllstoff umfasst, wobei der wasserlösliche Füllstoff ein Zucker ist.

## Revendications

1. Composition pharmaceutique sous forme de comprimé, comprenant a) une forme cristalline de bilastine, dans laquelle la forme cristalline présentant des pics caractéristiques à 6,53, 15,24, 15,86 et 18,07 ± 0,2 degrés 2-thêta

dans un diagramme de diffraction des rayons X sur poudre, b) un agent diluant soluble dans l'eau, et éventuellement c) un agent diluant insoluble dans l'eau, dans laquelle l'agent diluant soluble dans l'eau est un sucre, et dans laquelle la composition est préparée par granulation à sec d'un mélange des composants comprenant a), b) et éventuellement c), et compression des granulés dans un comprimé, ou compression directe d'un mélange des composants comprenant a), b) et éventuellement c).

**2.** Composition pharmaceutique selon la revendication 1, dans laquelle ladite composition est préparée par granulation à sec comprenant le mélange des composants de la revendication 1, le compactage au rouleau et la granulation du mélange, le broyage des granulés, la lubrification des granulés et la compression subsequente des granulés dans un comprimé.

**3.** Composition pharmaceutique selon la revendication 1 ou 2, dans laquelle la composition comprend en outre un agent désintégrant, un agent de glissement et / ou un agent lubrifiant.

**4.** Composition pharmaceutique selon l'une quelconque des revendications précédentes, dans laquelle l'agent diluant soluble dans l'eau est du mannitol.

**5.** Composition pharmaceutique selon l'une quelconque des revendications précédentes, dans laquelle l'agent diluant soluble dans l'eau est du lactose.

**6.** Composition pharmaceutique selon l'une quelconque des revendications précédentes, dans laquelle l'agent diluant insoluble dans l'eau est la cellulose microcristalline.

**7.** Composition pharmaceutique selon l'une quelconque des revendications précédentes, dans laquelle la composition comprend un agent désintégrant choisi parmi le glycolate d'amidon sodique, l'hydroxypropylcellulose faiblement substituée, l'amidon prégélatinisé ou la crospovidone, de préférence le glycolate d'amidon sodique ou la crospovidone.

**8.** Composition pharmaceutique selon l'une quelconque des revendications précédentes, dans laquelle la composition comprend un agent de glissement, qui est du dioxyde de silicium, de préférence sous forme de silice colloïdale anhydre, d'aluminométasilicate de magnésium et / ou de talc, de préférence une combinaison de dioxyde de silicium et d'aluminométasilicate de magnésium.

**9.** Composition pharmaceutique selon l'une quelconque des revendications précédentes, dans laquelle la composition comprend un agent lubrifiant, qui est du stéarate de magnésium.

**10.** Composition pharmaceutique selon l'une quelconque des revendications précédentes, comprenant une forme cristalline de bilastine, dans laquelle la forme cristalline présentant des pics caractéristiques à 6,53, 15,24, 15,86 et 18,07 $\pm$ 0,2 degrés 2-thêta dans un diagramme de diffraction des rayons X sur poudre, un agent diluant soluble dans l'eau, un agent diluant insoluble dans l'eau, un agent désintégrant, un agent de glissement et un agent lubrifiant, dans laquelle l'agent diluant soluble dans l'eau est le D-mannitol et l'agent de glissement est la silice colloïdale anhydre ou une combinaison de dioxyde de silicium et d'aluminométasilicate de magnésium.

**11.** Composition pharmaceutique selon la revendication 3, dans laquelle l'agent diluant soluble dans l'eau est le D-mannitol, l'agent diluant insoluble dans l'eau est la cellulose microcristalline, l'agent désintégrant est le glycolate d'amidon sodique, l'agent de glissement est la silice colloïdale anhydre ou une combinaison de silice colloïdale anhydre et aluminométasilicate de magnésium, et l'agent lubrifiant est le stéarate de magnésium.

**12.** Composition pharmaceutique selon l'une quelconque des revendications précédentes, dans laquelle le comprimé est un comprimé à libération immédiate enrobé ou non enrobé, de préférence non enrobé.

**13.** Composition pharmaceutique selon l'une quelconque des revendications précédentes, destinée à utiliser comme médicament dans le traitement de la rhino-conjonctivite allergique et / ou de l'urticaire.

**14.** Procédé de préparation d'une composition pharmaceutique sous la forme d'un comprimé, comprenant :

a. granulation à sec d'un mélange et compression des granulés dans un comprimé, dans laquelle le mélange comprend a) une forme cristalline de bilastine, dans laquelle la forme cristalline présentant des pics caractéristiques à 6,53, 15,24, 15,86 et 18,07 ± 0,2 degrés 2-thêta dans diagramme de diffraction des rayons X sur poudre, b) un agent diluant soluble dans l'eau et éventuellement c) un agent diluant insoluble dans l'eau, dans laquelle l'agent diluant soluble dans l'eau est un sucre, de préférence dans laquelle le procédé comprend le mélange des composants a), b) et éventuellement c), le compactage au rouleau et la granulation du mélange, broyage des granulés, lubrification des granulés et compression ultérieure des granulés dans un comprimé; ou
b. compression directe d'un mélange dans un comprimé, dans laquelle le mélange comprend a) une forme cristalline de bilastine, dans laquelle la forme cristalline présentant des pics caractéristiques à 6,53, 15,24, 15,86 et 18,07 ± 0,2 degrés 2-thêta dans une diffraction des rayons X sur poudre, b) un agent diluant soluble dans l'eau et éventuellement c) un agent diluant insoluble dans l'eau, dans laquelle l'agent diluant soluble dans l'eau est un sucre.

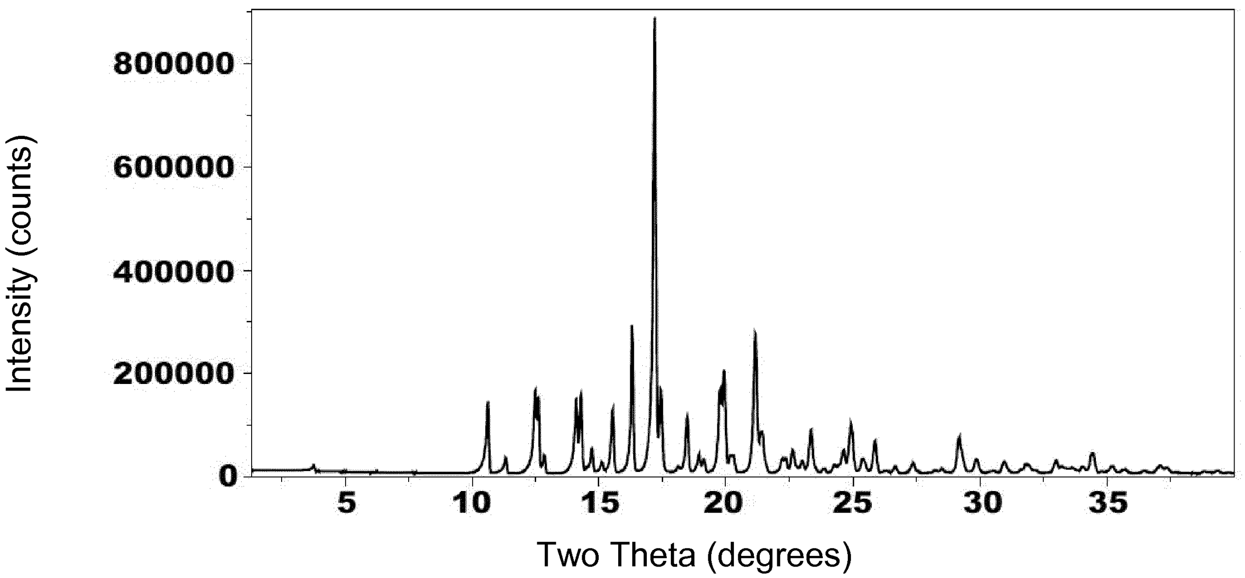
800000
600000
400000
200000
0
Intensity (counts)
5
10
15
20
25
30
35
Two Theta (degrees)

FIG. 1

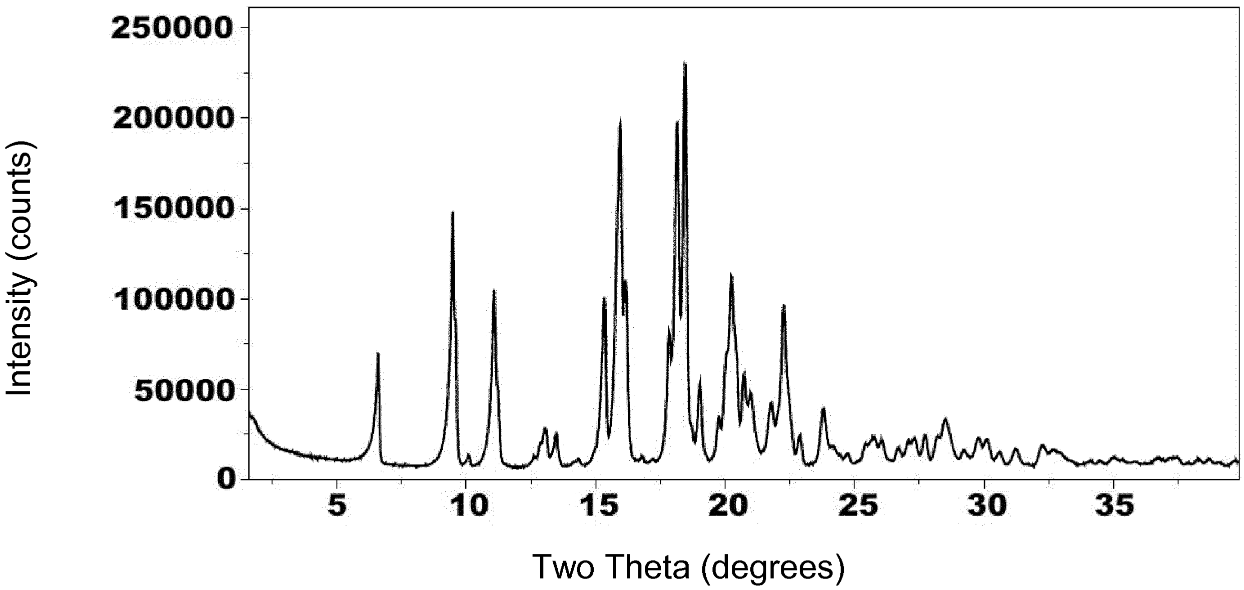

250000
200000
150000
100000
50000
0
Intensity (counts)
5
10
15
20
25
30
35
Two Theta (degrees)


FIG. 2

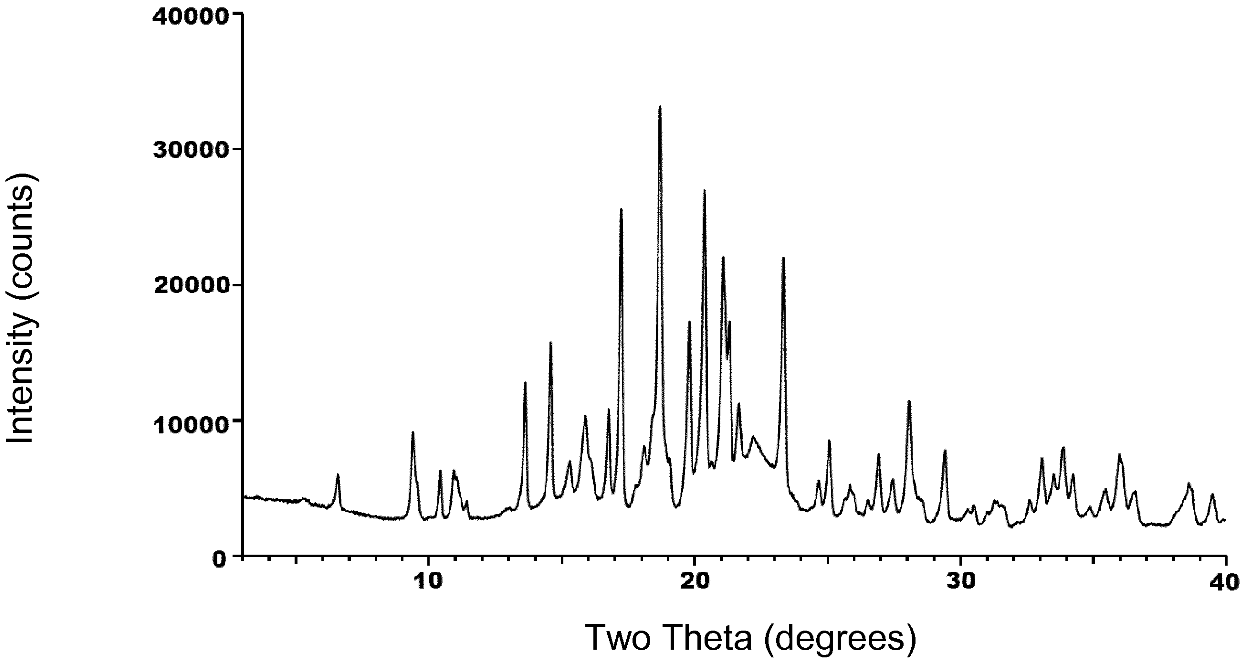
40000
30000
20000
10000
0
Intensity (counts)
10
20
30
40
Two Theta (degrees)

FIG. 3

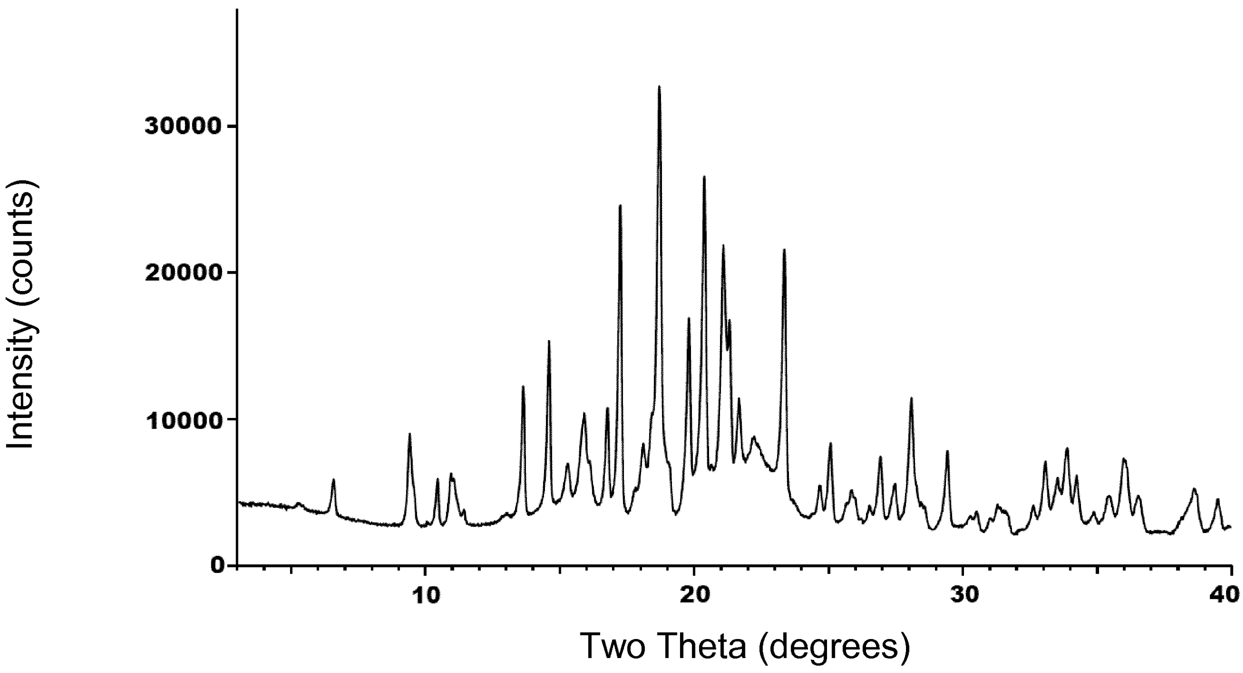
30000
20000
10000
0
10
20
30
40
Intensity (counts)
Two Theta (degrees)

FIG. 4

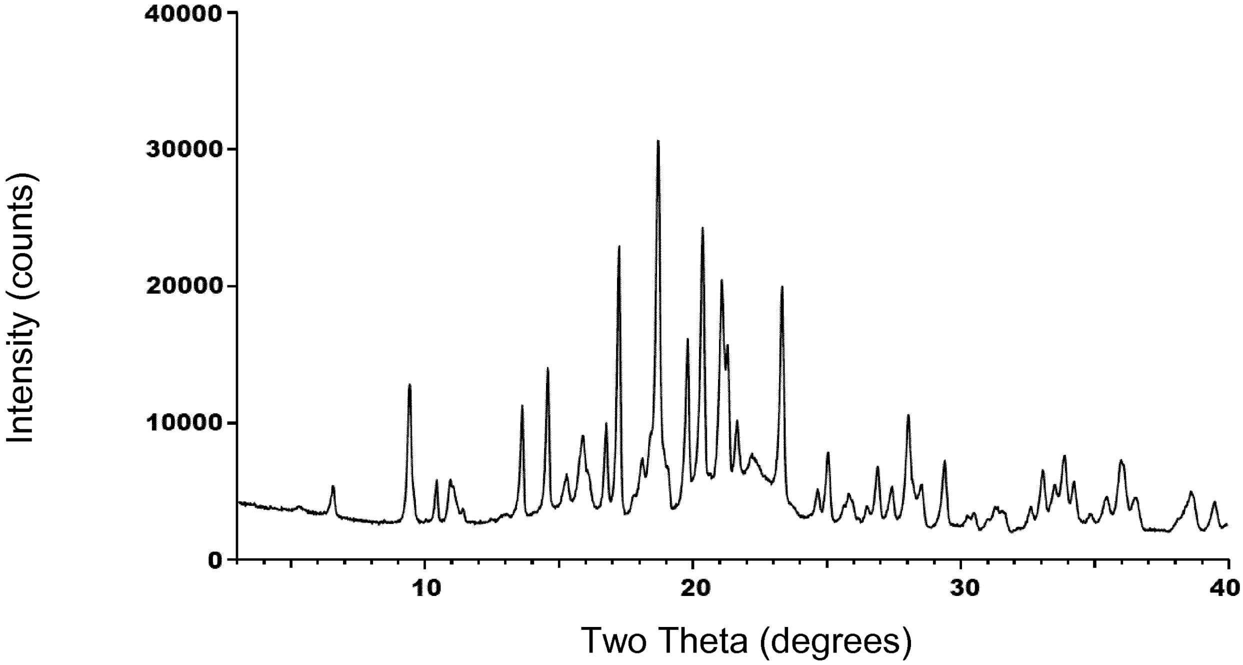
40000
30000
20000
10000
0
10
20
30
40
Intensity (counts)
Two Theta (degrees)

FIG. 5

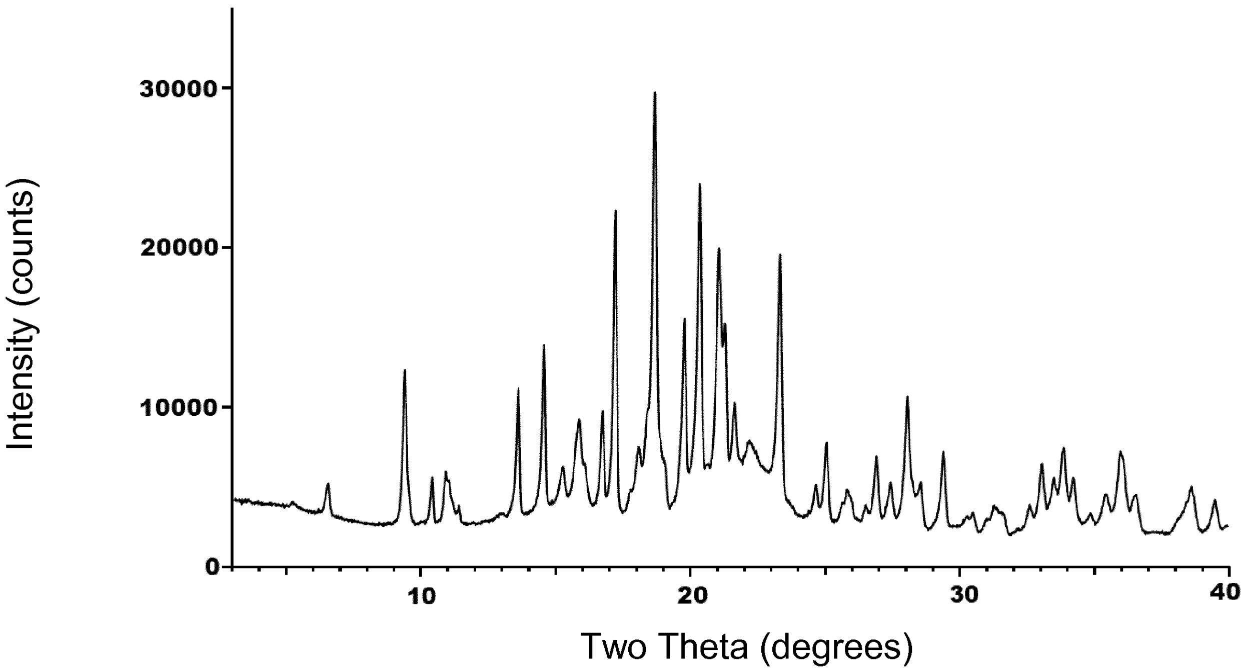
Intensity (counts)
30000
20000
10000
0
10
20
30
40
Two Theta (degrees)

**FIG. 6**

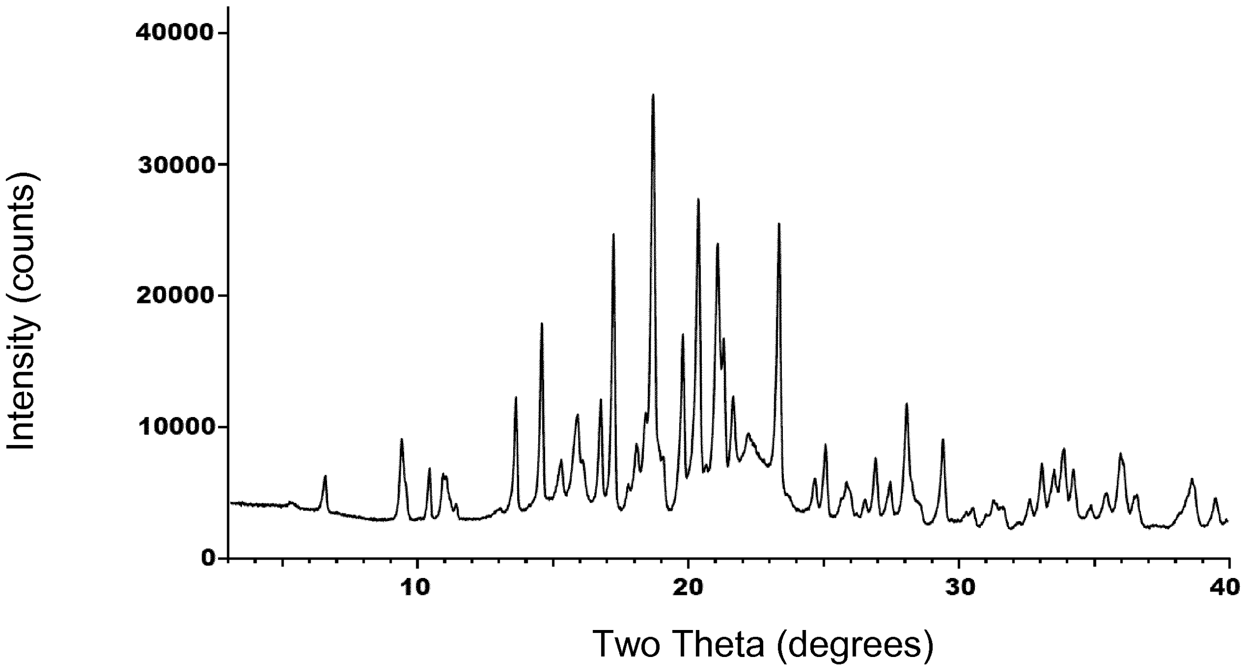
40000
30000
20000
10000
0
Intensity (counts)
10
20
30
40
Two Theta (degrees)

FIG. 7

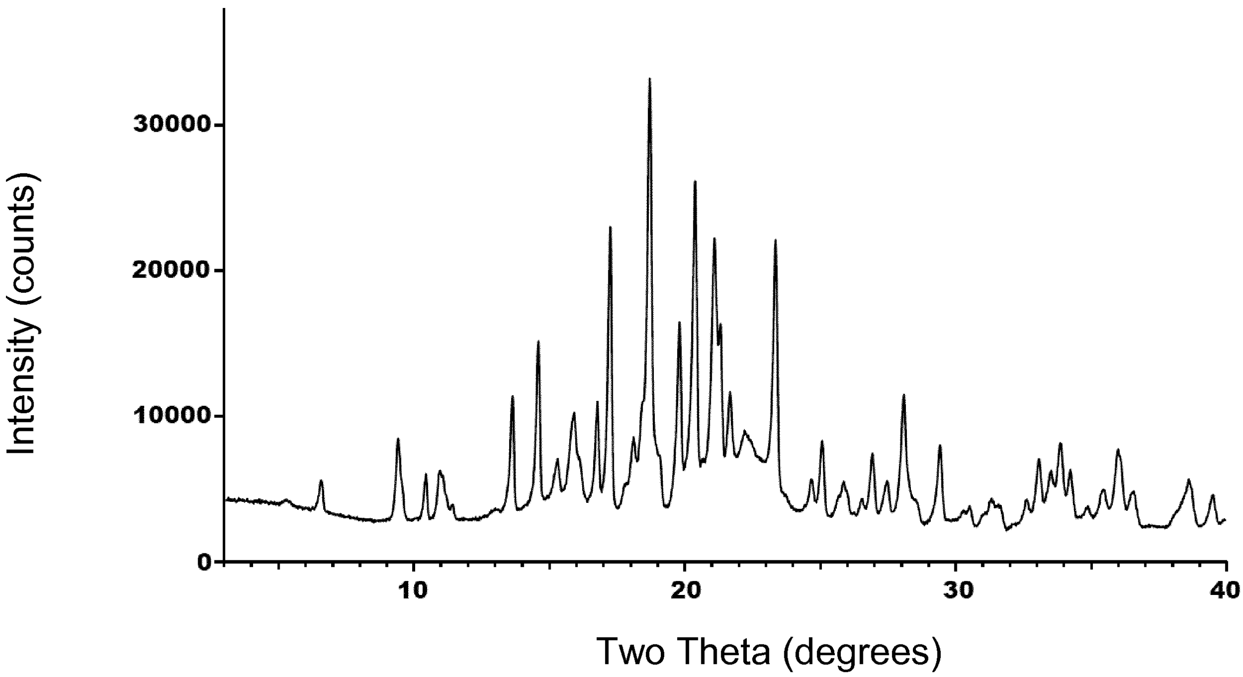
Intensity (counts)
30000
20000
10000
0
10
20
30
40
Two Theta (degrees)

FIG. 8

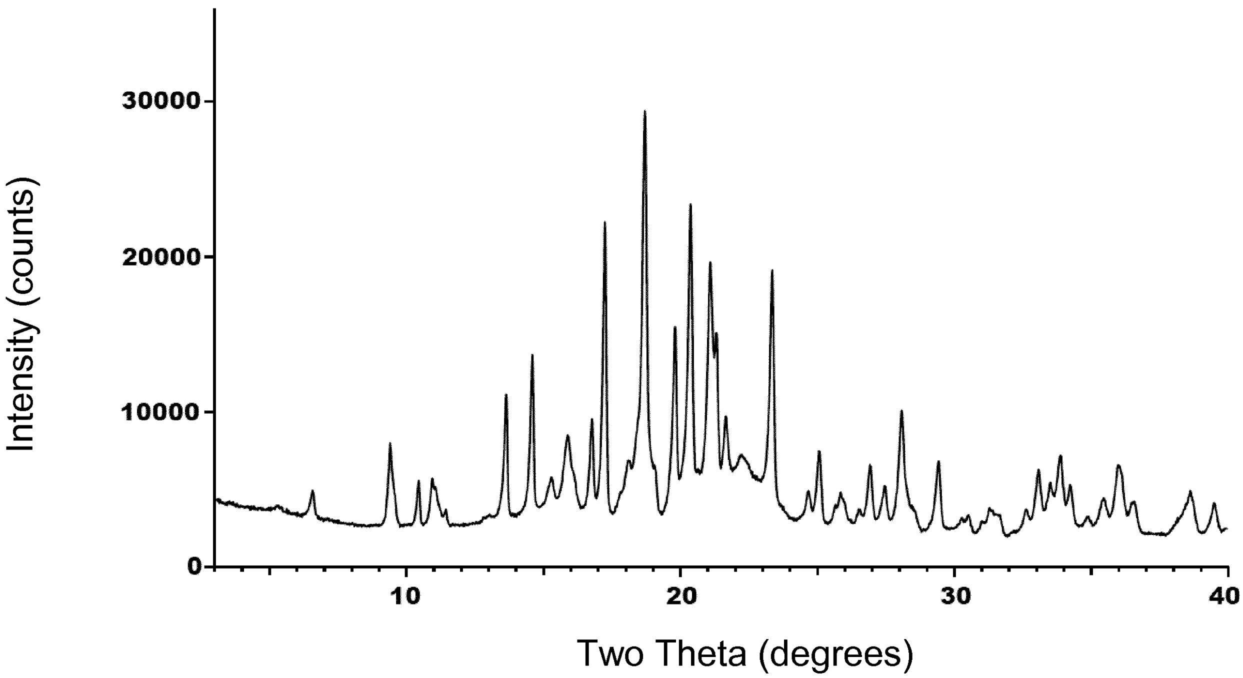
Intensity (counts)
30000
20000
10000
0
10
20
30
40
Two Theta (degrees)

**FIG. 9**

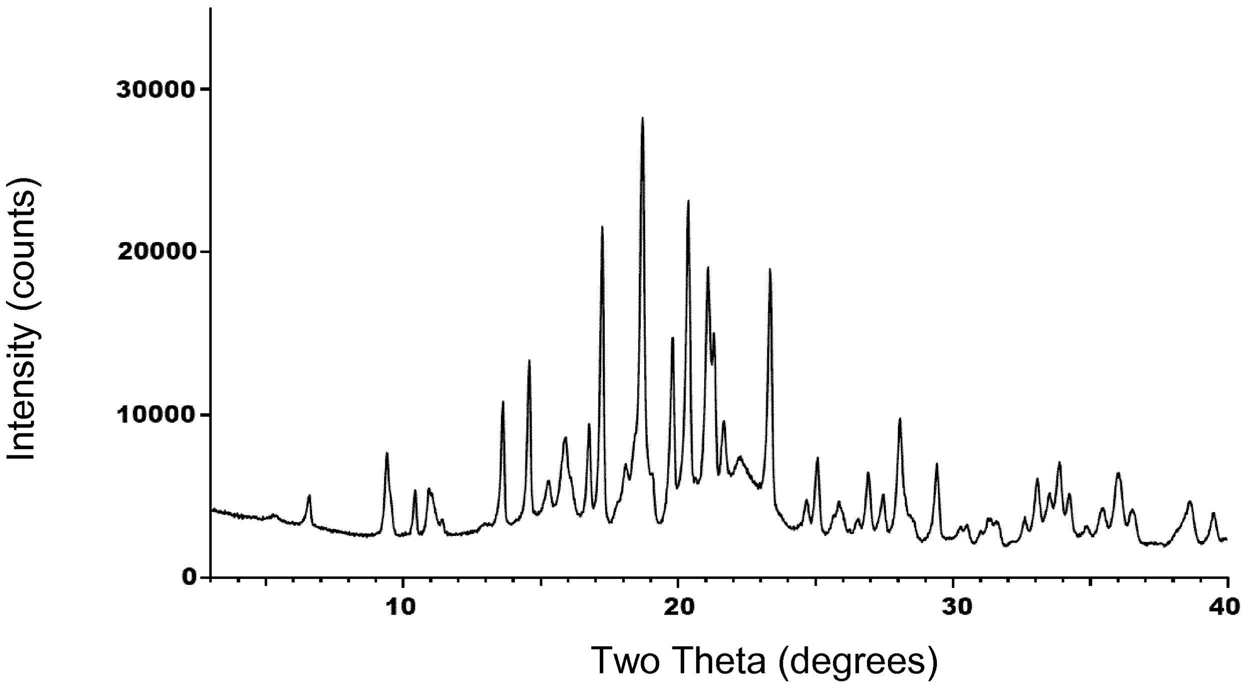
Intensity (counts)
30000
20000
10000
0
10
20
30
40
Two Theta (degrees)

FIG. 10

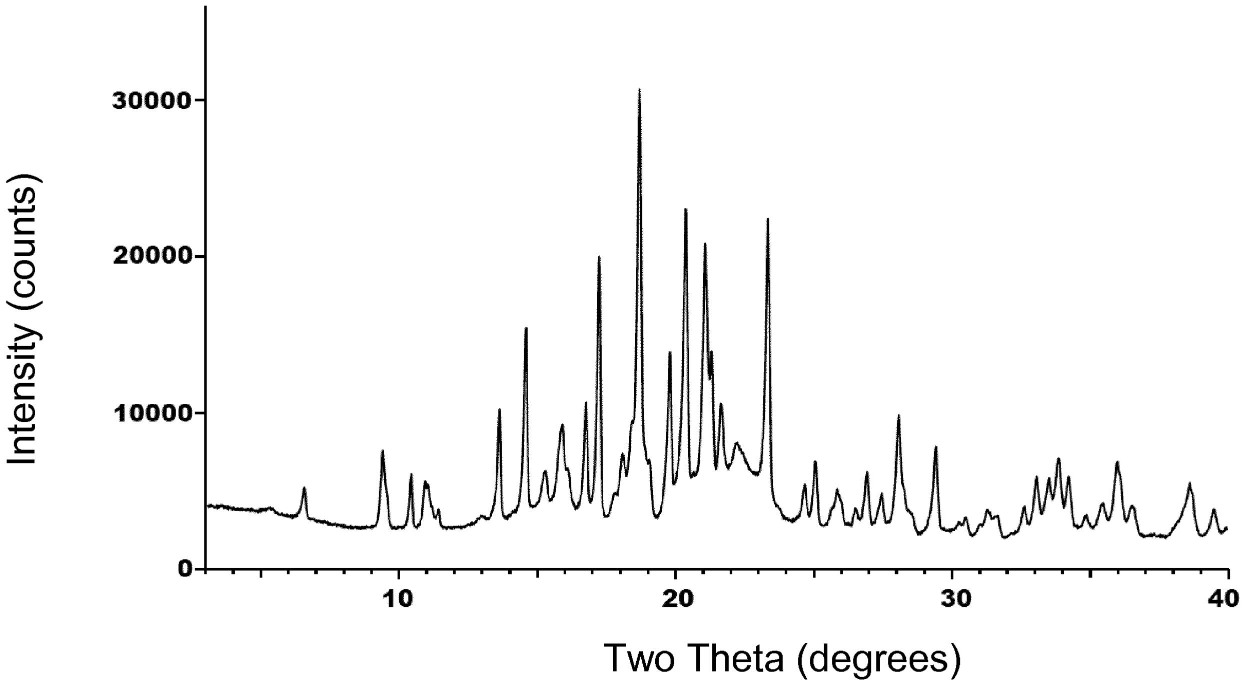
Intensity (counts)
30000
20000
10000
0
10
20
30
40
Two Theta (degrees)

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0818454 A
- EP 1505066 A
- EP 818454 A
- WO 2014026657 A
- WO 2017017301 A


### Non-patent literature cited in the description


- *CHEMICAL ABSTRACTS,* 202189-78-4
- *CHEMICAL ABSTRACTS,* 12511-31-8